# EUROPEAN PATENT APPLICATION

(11) **EP 3 388 447 A1**
(43) Date of publication of application: **17.10.2018**
(21) Application number: 17166696.9
(22) Date of filing: 14.04.2017
(51) Int. Cl.: C07K 14/435

(54) **METHODS TO PRODUCE PEPTIDES, POLYPEPTIDES OR CELLS FOR MODULATING IMMUNITY**

(71) Applicant: IMNATE SARL, 8041 Strassen (LU)
(72) Inventor: SAINT-REMY, Jean-Marie, 1390 Grez-Doiceau (BE)
(74) Representative: Office Kirkpatrick

(57) **Abstract**

The invention relates to a method to determine, based on the activation level of NKT cells, the capacity of a peptidic epitope to selectively bind to CD1a, CD1b and/or CD1c proteins, a corresponding method to increase or decrease the selective binding of the peptidic epitope to CD1a, CD1b and/or CD1c proteins by modifying the epitope or its flanking regions, the corresponding modified epitopes and the activated NKT cells or parts thereof obtainable by these methods.

SEQUENCE LISTING

<110>  Imnate SA-arl

<120>  METHODS TO PRODUCE PEPTIDES, POLYPEPTIDES OR CELLS FOR MODULATING
       IMMUNITY

<130>  Pbf1740EP00

<160>  21

<170>  PatentIn version 3.5

<210>  1
<211>  13
<212>  PRT
<213>  Homo sapiens

<220>
<221>  MISC_FEATURE
<223>  epitope p53

<400>  1

Lys Thr Tyr Gly Gly Ser Tyr Gly Phe Arg Leu Gly Phe
1               5                   10

<210>  2
<211>  16
<212>  PRT
<213>  Artificial Sequence

<220>
<223>  CD8 Binding

<400>  2

Lys Thr Tyr Gly Gly Ser Tyr Gly Phe Arg Leu Gly Phe Thr Gln Gln
1               5                   10                  15

<210>  3
<211>  10
<212>  PRT
<213>  Mycobacterium tuberculosis

<220>
<221>  MISC_FEATURE
<223>  Epitope M. Tuberculosis

<400>  3

Gln Gln Trp Asn Phe Ala Gly Ile Glu Ala
1               5                   10

<210>  4
<211>  8
<212>  DNA
<213>  Artificial Sequence

<220>
<223>  CpG ODN

<400>  4
acgttcgc                                                                 8


<210>  5
<211>  9
<212>  PRT
<213>  Homo sapiens


<220>
<221>  MISC_FEATURE
<223>  Epitope MOG protein

<400>  5

Phe Tyr Trp Val Ser Pro Gly Val Leu
1               5


<210>  6
<211>  12
<212>  PRT
<213>  Artificial Sequence

<220>
<223>  SEQ 5 + motif CD4

<400>  6

Phe Tyr Trp Val Ser Pro Gly Val Leu Arg Tyr Asp
1               5                   10


<210>  7
<211>  11
<212>  PRT
<213>  Homo sapiens

<400>  7

Trp Cys Gln Val Ala Gln Lys Phe Thr Gly Gly
1               5                   10


<210>  8
<211>  14
<212>  PRT
<213>  Artificial Sequence

<220>
<223>  SEQ 10 (GAD65) + CD4 motif

<400>  8

Trp Cys Gln Val Ala Gln Lys Phe Thr Gly Gly Arg Tyr Asp
1               5                   10

<210>  9
<211>  14
<212>  PRT
<213>  Artificial Sequence

<220>
<223>  SEQ 8 modified


<220>
<221>  LIPID
<222>  (2)..(2)
<223>  PALMITATE

<400>  9

Trp Cys Gln Val Ala Gln Lys Phe Thr Gly Gly Arg Tyr Asp
1               5                   10


<210>  10
<211>  8
<212>  PRT
<213>  Dermatophagoides pteronyssinus


<220>
<221>  MISC_FEATURE
<223>  DerP1

<400>  10

Tyr Trp Ile Val Arg Asn Ser Trp
1               5


<210>  11
<211>  5
<212>  PRT
<213>  Artificial Sequence

<220>
<223>  CXHX motif

<400>  11

Arg Cys Pro His Cys
1               5


<210>  12
<211>  13
<212>  PRT
<213>  Artificial Sequence

<220>
<223>  SEQs10+11

<400>  12

Tyr Trp Ile Val Arg Asn Ser Trp Arg Cys Pro His Cys
1               5                   10

<210>  13
<211>  11
<212>  PRT
<213>  Haemophilus influenzae

<400>  13

Phe Ile Asn Ala Gly Tyr Ile Phe Gln His His
1               5                   10


<210>  14
<211>  14
<212>  PRT
<213>  Artificial Sequence

<220>
<223>  SEQ 14 + Cys and Cys

<400>  14

His Cys Phe Ile Asn Ala Gly Tyr Ile Phe Gln His His Cys
1               5                   10


<210>  15
<211>  524
<212>  PRT
<213>  Homo sapiens


<220>
<221>  MISC_FEATURE
<223>  Pompe disease

<400>  15

Met Tyr Ala Leu Phe Leu Leu Ala Ser Leu Leu Gly Ala Ala Leu Ala
1               5                   10                  15


Gly Pro Val Leu Gly Leu Lys Glu Cys Thr Arg Gly Ser Ala Val Trp
            20                  25                  30


Cys Gln Asn Val Lys Thr Ala Ser Asp Cys Gly Ala Val Lys His Cys
        35                  40                  45


Leu Gln Thr Val Trp Asn Lys Pro Thr Val Lys Ser Leu Pro Cys Asp
    50                  55                  60


Ile Cys Lys Asp Val Val Thr Ala Ala Gly Asp Met Leu Lys Asp Asn
65                  70                  75                  80


Ala Thr Glu Glu Glu Ile Leu Val Tyr Leu Glu Lys Thr Cys Asp Trp
                85                  90                  95


Leu Pro Lys Pro Asn Met Ser Ala Ser Cys Lys Glu Ile Val Asp Ser

100                 105                 110

Tyr Leu Pro Val Ile Leu Asp Ile Ile Lys Gly Glu Met Ser Arg Pro
        115                 120                 125

Gly Glu Val Cys Ser Ala Leu Asn Leu Cys Glu Ser Leu Gln Lys His
    130                 135                 140

Leu Ala Glu Leu Asn His Gln Lys Gln Leu Glu Ser Asn Lys Ile Pro
145                 150                 155                 160

Glu Leu Asp Met Thr Glu Val Val Ala Pro Phe Met Ala Asn Ile Pro
                165                 170                 175

Leu Leu Leu Tyr Pro Gln Asp Gly Pro Arg Ser Lys Pro Gly Pro Lys
            180                 185                 190

Asp Asn Gly Asp Val Cys Gln Asp Cys Ile Gln Met Val Thr Asp Ile
        195                 200                 205

Gln Thr Ala Val Arg Thr Asn Ser Thr Phe Val Gln Ala Leu Val Glu
    210                 215                 220

His Val Lys Glu Glu Cys Asp Arg Leu Gly Pro Gly Met Ala Asp Ile
225                 230                 235                 240

Cys Lys Asn Tyr Ile Ser Gln Tyr Ser Glu Ile Ala Ile Gln Met Met
                245                 250                 255

Met His Met Gln Pro Lys Glu Ile Cys Ala Leu Val Gly Phe Cys Asp
            260                 265                 270

Glu Val Lys Glu Met Pro Met Gln Thr Leu Val Pro Ala Lys Val Ala
        275                 280                 285

Ser Lys Asn Val Ile Pro Ala Leu Glu Leu Val Glu Pro Ile Lys Lys
    290                 295                 300

His Glu Val Pro Ala Lys Ser Asp Val Tyr Cys Glu Val Cys Glu Phe
305                 310                 315                 320

Leu Val Lys Glu Val Thr Lys Leu Ile Asp Asn Asn Lys Thr Glu Lys
                325                 330                 335

Glu Ile Leu Asp Ala Phe Asp Lys Met Cys Ser Lys Leu Pro Lys Ser
            340                 345                 350

Leu Ser Glu Glu Cys Gln Glu Val Val Asp Thr Tyr Gly Ser Ser Ile
        355                 360                 365

Leu Ser Ile Leu Leu Glu Glu Val Ser Pro Glu Leu Val Cys Ser Met
    370                 375                 380

Leu His Leu Cys Ser Gly Thr Arg Leu Pro Ala Leu Thr Val His Val
385                 390                 395                 400

Thr Gln Pro Lys Asp Gly Gly Phe Cys Glu Val Cys Lys Lys Leu Val
                405                 410                 415

Gly Tyr Leu Asp Arg Asn Leu Glu Lys Asn Ser Thr Lys Gln Glu Ile
            420                 425                 430

Leu Ala Ala Leu Glu Lys Gly Cys Ser Phe Leu Pro Asp Pro Tyr Gln
        435                 440                 445

Lys Gln Cys Asp Gln Phe Val Ala Glu Tyr Glu Pro Val Leu Ile Glu
    450                 455                 460

Ile Leu Val Glu Val Met Asp Pro Ser Phe Val Cys Leu Lys Ile Gly
465                 470                 475                 480

Ala Cys Pro Ser Ala His Lys Pro Leu Leu Gly Thr Glu Lys Cys Ile
                485                 490                 495

Trp Gly Pro Ser Tyr Trp Cys Gln Asn Thr Glu Thr Ala Ala Gln Cys
            500                 505                 510

Asn Ala Val Glu His Cys Lys Arg His Val Trp Asn
        515                 520

<210>  16
<211>  524
<212>  PRT
<213>  Artificial Sequence

<220>
<223>  Mutated pompe

<400>  16

Met Tyr Ala Leu Phe Leu Leu Ala Ser Leu Leu Gly Ala Ala Leu Ala
1               5                   10                  15

Gly Pro Val Leu Gly Leu Lys Glu Cys Thr Arg Gly Ser Ala Val Trp
            20                  25                  30

Cys Gln Asn Val Lys Thr Ala Ser Asp Cys Gly Ala Val Lys His Cys

        35                  40                  45

Leu Gln Thr Val Trp Asn Lys Pro Thr Val Lys Ser Leu Pro Cys Asp
    50                  55                  60

Ile Cys Lys Asp Val Val Thr Ala Ala Gly Asp Met Leu Lys Asp Asn
65                  70                  75                  80

Ala Thr Glu Glu Glu Ile Leu Val Ser Leu Glu Lys Thr Cys Asp Ala
                85                  90                  95

Leu Pro Lys Pro Asn Met Ser Ala Ser Cys Lys Glu Ile Val Asp Ser
            100                 105                 110

Tyr Leu Pro Val Ile Leu Asp Ile Ile Lys Gly Glu Met Ser Arg Pro
        115                 120                 125

Gly Glu Val Cys Ser Ala Leu Asn Leu Cys Glu Ser Leu Gln Lys His
    130                 135                 140

Leu Ala Glu Leu Asn His Gln Lys Gln Leu Glu Ser Asn Lys Ile Pro
145                 150                 155                 160

Glu Leu Asp Met Thr Glu Val Val Ala Pro Phe Met Ala Asn Ile Pro
                165                 170                 175

Leu Leu Leu Tyr Pro Gln Asp Gly Pro Arg Ser Lys Pro Gly Pro Lys
            180                 185                 190

Asp Asn Gly Asp Val Cys Gln Asp Cys Ile Gln Met Val Thr Asp Ile
        195                 200                 205

Gln Thr Ala Val Arg Thr Asn Ser Thr Phe Val Gln Ala Leu Val Glu
    210                 215                 220

His Val Lys Glu Glu Cys Asp Arg Leu Gly Pro Gly Met Ala Asp Ile
225                 230                 235                 240

Cys Lys Asn Tyr Ile Ser Gln Tyr Ser Glu Ile Ala Ile Gln Met Met
                245                 250                 255

Met His Met Gln Pro Lys Glu Ile Cys Ala Leu Val Gly Phe Cys Asp
            260                 265                 270

Glu Val Lys Glu Met Pro Met Gln Thr Leu Val Pro Ala Lys Val Ala
        275                 280                 285

Ser Lys Asn Val Ile Pro Ala Leu Glu Leu Val Glu Pro Ile Lys Lys
    290                 295                 300

His Glu Val Pro Ala Lys Ser Asp Val Ser Cys Glu Val Cys Glu Val
305                 310                 315                 320

Leu Val Lys Glu Val Thr Lys Leu Ile Asp Asn Asn Lys Thr Glu Lys
                325                 330                 335

Glu Ile Leu Asp Ala Phe Asp Lys Met Cys Ser Lys Leu Pro Lys Ser
            340                 345                 350

Leu Ser Glu Glu Cys Gln Glu Val Val Asp Thr Tyr Gly Ser Ser Ile
        355                 360                 365

Leu Ser Ile Leu Leu Glu Glu Val Ser Pro Glu Leu Val Cys Ser Met
    370                 375                 380

Leu His Leu Cys Ser Gly Thr Arg Leu Pro Ala Leu Thr Val His Val
385                 390                 395                 400

Thr Gln Pro Lys Asp Gly Gly Phe Cys Glu Val Cys Lys Lys Leu Val
                405                 410                 415

Gly Tyr Leu Asp Arg Asn Leu Glu Lys Asn Ser Thr Lys Gln Glu Ile
            420                 425                 430

Leu Ala Ala Leu Glu Lys Gly Cys Ser Phe Leu Pro Asp Pro Tyr Gln
        435                 440                 445

Lys Gln Cys Asp Gln Phe Val Ala Glu Tyr Glu Pro Val Leu Ile Glu
    450                 455                 460

Ile Leu Val Glu Val Met Asp Pro Ser Phe Val Cys Leu Lys Ile Gly
465                 470                 475                 480

Ala Cys Pro Ser Ala His Lys Pro Leu Leu Gly Thr Glu Lys Cys Ile
                485                 490                 495

Trp Gly Pro Ser Tyr Trp Cys Gln Asn Thr Glu Thr Ala Ala Gln Cys
            500                 505                 510

Asn Ala Val Glu His Cys Lys Arg His Val Trp Asn
        515                 520

<210>  17
<211>  13
<212>  PRT

<213>  Artificial Sequence

<220>
<223>  AAV

<400>  17

Phe Asn Gly Leu Asp Lys Gly Glu Pro Val Asn Glu Ala
1               5                   10


<210>  18
<211>  17
<212>  PRT
<213>  Artificial Sequence

<220>
<223>  SEQ 17 + Cys and Cys

<400>  18

His Cys Phe Asn Gly Leu Asp Lys Gly Glu Pro Val Asn Glu Ala Cys
1               5                   10                  15


His




<210>  19
<211>  12
<212>  PRT
<213>  Artificial Sequence

<220>
<223>  Celiac Disease

<400>  19

Gln Leu Gln Pro Phe Pro Gln Pro Gln Leu Pro Tyr
1               5                   10


<210>  20
<211>  17
<212>  PRT
<213>  Artificial Sequence

<220>
<223>  SEQ I9 + CD4 motif

<400>  20

Gln Leu Gln Pro Phe Pro Gln Pro Gln Leu Pro Tyr Pro Gln Arg Tyr
1               5                   10                  15


Asp




<210>  21
<211>  14

<212>  PRT
<213>  Artificial Sequence

<220>
<223>  Flavivirus NS1 + CD8 motif

<400>  21

Phe Ile Glu Val Lys Ser Cys His Trp Ser Asn Thr Gln Gln
1               5                   10

## Description

### Field of invention

The present invention relates to methods to produce peptides encompassing epitopes activating natural killer T (NKT) cells and their use in treating conditions wherein it is advantageous to elicit an innate immune response, such as in tumors, infectious diseases, chronic autoimmune and inflammatory diseases, and immunosenescence.

The invention also relates to methods to produce peptides or polypeptides having lost their capacity to activate NKT cells for use in situations wherein alloantigens have to be administered for therapeutic purposes or to which individuals are exposed by food. Further, the present invention relates to peptides modified so as to suppress the immunogenicity of said alloantigens.

The invention also relates to cells engineered to carry a chimeric receptor for the therapy of tumors and autoimmune diseases.

### Background of the invention

Innate immunity is considered as non-specific in the sense that it is elicited by recognition of the offending agent by receptors specific for patterns associated with extrinsic or intrinsic molecules. Thus, pathogen-associated molecular patterns (PAMPs) bind and activate receptors such as Toll-like receptors (TLRs), and molecules produced by, for instance, oxidative stress bind and activate damage-associated molecular patterns (DAMPs).

Cells of the NKT lineage, which are classified as part of innate immunity, do in fact occupy an intermediate position in between innate and adaptive responses, in so far as NKT cells express an antigen-specific receptor (CD3) capable of forming a cognate synapse with an antigen-presenting cell expressing the MHC-like CD1 molecule acting as a restriction element. NKT cells recognize and are activated by lipids or glycolipids bound to the CD1 molecule.

The paradigm of such activation is represented by NKT cells carrying an invariant alpha chain as part of the CD3 receptor, and are activated by presentation of a glycolipid (alpha-gal-ceramide) via presentation in association with a CD1 molecule, CD1d, which is the only constituent of a distinct group (group 2) of CD1 molecules. Thus, the conventional view on NKT cells includes almost exclusively invariant NKT (iNKT) or type 1 NKT cells, activated by alpha-gal-ceramide in the context of presentation by group 2 CD1d molecule (see reviews, such as Matsuda et al, Current Opinion in Immunology 2008, 20:358-368 and Godfrey et al, Nature reviews Immunology 2010, 11: 197-206). A less defined type 2 NKT cells, carrying diverse CD3 alpha chains, is also distinguished, with specificity for lipids or glycolipids presented by group 2 CD1d molecule.

A distinct group of CD1 molecules, referred to as group 1 CD1 and including CD1a, CD1b, CD1c and CD1e molecules, is expressed in humans but not in the mouse. The CD1 locus is encoded on chromosome 1. Such group 1 CD1 molecules participate to cell surface screening, intracellular trafficking and presentation at cell surface of a number of lipids or glycolipids derived from infectious agents or from endogenous source. Best examples of this are sulfatides and products derived from cells infected with *Mycobacterium tuberculosis* (Felio et al, Journal of Experimental Medicine, 2009, 206:2497-2509). They greatly differ from each other, and from CD1d, by their structure, distribution, regulation, intracellular trafficking, molecular interaction and function. CD1e is confined to the intracellular environment, whilst CD1a, CD1b and CD1c are expressed at the surface of cells and, as such, can interact directly with cells of the NKT lineage.

In all of the above-cited prior art, NKT cells are deemed to recognize and be activated only by lipids or glycolipids, and not by peptidic sequences.

However, patent applications WO2012069568, WO2012069572, WO2012069575 and WO2013174805 describe a number of CD1d-restricted peptidic epitopes from various antigens, both intrinsic, such as GAD65, and extrinsic, such as those from viral vectors. Such epitopes can be used to increase activation of specific NKT cells, in particular when combined to an oxido-reductase motif within flanking residues (WO2012069568), or by increasing the binding of the core sequence of the epitope by introducing single aminoacid residue mutation (WO2012069572). Likewise, patent application WO2012069575 describes the elimination of CD1d-restricted epitopes from therapeutic proteins so as to prevent undesirable activation of NKT cells, with patent application WO2013174805 describing methods to reduce CD1d-restricted activation of NKT cell elicited by administration of coagulation factor VIII for the treatment of hemophilia A.

Peptidic epitope-specific NKT cells as described in the above patent applications are restricted by CD1d, representative of group 2 MHC-like restriction molecules.

Huge unmet medical needs still exist in a number of pathological conditions and, prominently, for the treatment of tumors and for infections with intracellular pathogens.

Despite significant progress made in the therapy of tumors, including therapeutic antibodies targeting checkpoint inhibitors and chimeric antigen receptor (CAR) T cells, the efficacy of these approaches is limited to a minority of patients with no possibility to predict which patient will be responsive. The complexity of the tumor microenvironment, its versatility, the number of cells involved and the general immunosuppressive conditions in such microenvironment makes efficient and predictable therapy a far from being reached goal.

Tumors share many metabolic abnormalities, which could constitute an appropriate and safe target for the design of a therapy, which would spare other tissues. Unfortunately, most tumor cells downregulate surface expression of molecules, including those of major histocompatibility complexes (MHC), thereby becoming invisible to adaptive immune responses. In addition, complex intra- and peri-tumor regulatory circuits emerge, which neutralize the immune system attempts to eliminate tumor cells.

Intracellular pathogens include viral diseases, bacteria, mycobacteria, and parasites. Most of these pathogens elaborate strategies which neutralize the specific adaptive response by diverting components of the MHC machinery, adaptor proteins or pathways involved in the activation of infected cells. Infected cells thereby become invisible to cells participating to the adaptive immune response. However, with some exceptions, such as for type 1 and 2 herpes virus, infected cells keep their capacity to present antigens at their surface in association with CD1 molecules. It is indeed well known that, for example, macrophages infected with mycobacterium tuberculosis express at their surface lipids bound to CD1 molecules.

Huge unmet medical needs also exist in a number of pathological conditions and in particular in the treatment of chronic autoimmune diseases and chronic inflammatory diseases.

Chronic diseases non associated with tumor or infection comprise both an autoimmune and an inflammatory component. As such, tissue inflammation releases antigens to which no tolerance is acquired because of lack of physiological exposure in thymus, or which are sufficiently modified by inflammation as to become new antigens. Autoimmune responses contribute to inflammation thereby creating a positive feedback mechanism maintaining the disease process.

This intricate pathogenesis *de facto* limits all therapeutic attempts to non-specific measures, such as, for example, anti-inflammatory drugs or therapeutic antibodies dampening the immune system or limiting access of cells to the affected organs. If the initiating antigen or at least one of the antigens directly implicated in pathology could be identified, then it would become feasible to develop antigen-specific approaches by which the overall disease process could be maintained under control. There have been attempts to suppress complex immune responses. However, in all these instances the chronic autoantigen specific inflammatory component, which elicits and maintains the autoimmune pathological response, is essentially left unaffected.

There is therefore a dramatic unmet medical need in the treatment of chronic autoimmune diseases, including type 1 diabetes, multiple sclerosis, rheumatoid arthritis, autoimmune thyroiditis and celiac disease, to cite just a few. Eliciting NKT cells with regulatory properties would represent an antigen-specific method to reduce inflammation and the autoimmune response.

In chronic asthma and in chronic obstructive or non obstructive pulmonary diseases, many antigens trigger and maintain chronic inflammation. Allergens such as those from the house dust mite represent one of the leading cause of allergic asthma. Chronic infection such as *Haemophilus influenzae*, or immune responses to elastin in chronic obstructive pulmonary diseases, represent sources of antigens triggering an inflammatory reaction. Reducing inflammation by eliciting NKT cells with regulatory properties would represent a method to reduce inflammation and therefore local tissue destruction.

Conditions associated with endoplasmic reticulum stress are numerous and left without specific treatment. Apart from chronic autoimmune diseases, tumors or chronic intracellular infections, the mechanisms leading to cell immunosenescence are also accompanied by such stress conditions.

In many instances, administration of proteins for therapeutic purposes results in immune response against the therapeutic agent, which precludes any further administration of said therapeutic agent. Examples of this are provided by administration of factor VIII of the coagulation pathway in subjects affected by hemophilia A: about a third of treated subjects produce anti-factor VIII antibodies inhibiting the function of factor VIII. Administration of enzymes such as acid alpha-glucosidase in subjects suffering from Pompe's disease is likewise followed by an immune response against the therapeutic agent precluding any further administration. A third example is provided by antibodies used as therapeutic agents and directed towards lymphocyte surface molecule, cytokines or cytokine receptors. Overall, it is highly desirable to find new therapeutic approaches which would prevent immunization against therapeutic agents.

The use of viral vectors for gene therapy or gene vaccination is severely restricted by the fact that such viral vectors elicit an immune response, which results in rapid elimination of cells transduced by the vector and rapid loss of transgene expression. Viral vectors elicit activation of the innate and adaptive immune responses with variable degree of intensity depending of the nature of the viral vector. Thus, adenovirus vectors strongly stimulate innate immune responses followed by an adaptive immune response. Adeno-associated viral vectors trigger a weaker innate response but elicit antibody production. The potential of both gene therapy and gene vaccination is huge. A therapeutic approach by which the innate and/or the adaptive response towards viral vectors is prevented would represent a highly significant progress in the field.

Many subjects suffer from response elicited against proteins to which they are naturally exposed. Allergens, either airborne or from food, elicit reactions such as allergic rhinitis and asthma, urticaria, eczema and anaphylactic reaction. The reason as to why said subjects present such reactions is only partially elucidated. It would be of much benefit to reduce the innate immunogenicity of proteins as diverse as food allergens, wheat causing celiac disease or products such as enzymes to which subjects are exposed for professional reasons.

CAR T cell efficacy, in particular in the treatment of tumors, remains limited both by the functional properties of the T cell, and by the specificity of the target molecule expression. The cytotoxic properties of NKT cells would represent a distinct advantage in the first case and CAR NKT cells carrying antibody variable parts towards a surface antigen of a target cell would provide improved efficacy in eradicating such target cell. In the second case, the exquisite specificity of cognate recognition of a CD1-bound epitope by NKT cells provides the possibility to produce NKTCR T cells, wherein a NKT antigen receptor is used to transfect cells of alternative lineage, making use of specific properties of such alternative T cells to target only cells expressing CD1-bound epitopes.

### Summary of the invention

The present invention relates to a method to determine the capacity of an epitope (an hydrophobic epitope) to selectively bind to CD1a, CD1b and/or CD1c proteins comprising the steps of:
a) providing isolated cells expressing at their surface CD1a, CD1b and/or CD1c;
b) (selectively) applying the epitope (to be tested) to the isolated cells;
c) putting NKT cells into contact with the cells of step b potentially presenting the epitope;
d) and measuring the activation level of the NKT cells.
Preferably, this method further comprises a step e) of isolating the activated NKT cells of step d).
Advantageously, the isolated NKT cells produce interferon gamma or exert a cytotoxic activity.
A closely related aspect of the present invention is thus the isolated NKT cells (of step e), the T cell receptor of such isolated NKT cell or the nucleic acid molecule encoding the T cell receptor of such isolated NKT, for use as a medicament, preferably in the treatment of cancer, or of diseases caused by an intracellular pathogen, or of auto immune disease (especially NKT cells that do not exert a cytotoxic activity). For instance, the nucleic acid encoding the T cell receptor can be subsequently used for the generation of CAR NKT cells.
Possibly, the present method further comprises the step of measuring the selective binding of the hydrophobic epitope on CD1d protein.
Advantageously, the isolated cells expressing at their surface CD1a, CD1b and/or CD1c are transformed cells to specifically over express these CD1a, CD1b and/or CD1c proteins. Advantageously, the isolated cells expressing at their surface CD1a, CD1b and/or CD1c are cancerous cells, cells targeted by auto-immune diseases, cells chronically exposed to an allergen or cells infected with an intracellular pathogen.
Possibly, the (hydrophobic) epitope is a domain from 7 to 20 consecutive amino acid having hydrophobic properties, for instance more than 20% (preferably, more than 25%) of the amino acids are hydrophobic (aromatic amino acid, methionine, valine, leucine, isoleucine, citrulline, lipid-modified amino acid such as cysteine) and/or hydrophobic amino acids are organized so as to display an hydrophobic moment.
Advantageously, the epitope is an isolated protein (or a fragment thereof) or a synthetic peptide comprising the amino acid sequence of a protein from a tumor, an intracellular pathogen, a biological protein, an allergen, an auto antigen or an agent causing chronic inflammation.
The present method is especially adapted for the screening of the (hydrophobic) epitope(s) comprising, or potentially comprising, post-translationally modified amino acids, preferably wherein the hydrophobicity of the said post-translationally modified amino acid is higher than the corresponding non modified amino acid (i.e. acylation, decarboxylation).
Possibly, in this method, the epitope is not the CD1d epitope [FYWTH]X₂X_{3[}ILMV]X₅X₆[FYWTH].

Possibly, the present method further comprises the step of selecting (hydrophobic) epitope(s) determined as to selectively bind to CD1a, CD1b and/or CD1c, preferably not binding to CD1d. Preferably, the isolated cells expressing at their surface CD1a, CD1b and/or CD1c have an expression level of the said CD1a, CD1b and/or CD1c higher than CD1d, more preferably these isolated cells do not express MHC class II molecules.
Advantageously, the NKT cells are CD4+ (with thus none, or only a low amount of NKT CD8+ and of NKT CD4- CD8-; i.e. more than 75% of the NKT cells are CD4+, preferably, more than 90%, still more preferably more than 95%), CD8+, (with thus none or only a low amount of NKT CD4+ and, possibly, none or only a low amount of NKT CD4- CD8-; i.e. more than 75% of the NKT cells are CD8+, preferably, more than 90%, still more preferably more than 95%) preferably αα or ββ, or CD4-/CD8-. A related aspect is a peptide obtainable by the present method for use in vaccination against cancer, against infections with intracellular pathogens, against auto-immune diseases or against allergic diseases, preferably this peptide comprises one or more of an acylated amino acid or of a decarboxylated amino acid.

Another related aspect of the present invention is a method to increase the capacity of an (hydrophobic) epitope (preferably a peptidic epitope) to bind to CD1a, CD1b and/or CD1c proteins comprising the steps of
a) providing isolated cells expressing at their surface CD1a, CD1b and/or CD1c;
b) (specifically) applying this epitope to these isolated cells;
c) putting NKT cells into contact with the cells of step b;
d) measuring a low or no activation of these NKT cells;
e) adding to this epitope a potentiating motif selected from the group consisting of hydrophobic amino acids, an hydrophobic post-translationally modified amino acid residue and/or deleting a non-hydrophobic amino acid from this epitope, so as to generate a modified epitope,
f) applying the modified epitope to these isolated cells;
g) putting NKT cells into contact with the cells of step f, potentially presenting the modified epitope and
h) selecting a modified epitope allowing an increased activation of the NKT cells in step g, by comparison with the measure of step d.
Preferably, the NKT cells are CD8+ or CD4-/CD8- (i.e. no CD4+ NKT cells), advantageously allowing to generate epitopes and cells leading to an increased cytotoxic response.
Preferably, this method further comprises, (before the step g) the step of adding in one or both flanking region(s) of the epitope a palmitoyl-cysteine moiety, a CD8-specific binding moiety (such as a TQQ tripeptide), a Toll-like-receptor (TLR) agonist (preferably a CpG oligonucleotide) and/or to add a cysteine amino acid in both flanking regions (preferably in the format of a cysteine motif His-Cys-x-epitope-x-C-H, where H stands for histidine, and X is from 0 to 7 not defined amino acids) of this epitope.
Possibly, the epitope is derived from cancerous cells or from cells infected with an intracellular pathogen.
Preferably, this method further comprises a step i) of isolating the activated NKT cells of step h).

A related aspect of the present invention is a peptide obtainable by this method for use in the treatment of vaccination against the cancer or the intracellular pathogen.
Alternatively, in this method to increase the capacity of an (hydrophobic) epitope to bind to CD1a, CD1b and/or CD1c proteins, the NKT cells are CD4+ (i.e. no CD8+ NKT cells and no CD4-/CD8- NKT cells), which preferably allows to generate strong regulatory epitopes and/or NKT cells.
Thus in this aspect, the method advantageously further comprises (before the step g) the step of adding in one or both flanking region(s) of the epitope a palmitoyl-cysteine moiety, a CD4-specific binding moiety (such as RYD tripeptide), and/ or to add a cysteine amino acid (or motif, as described above)in both flanking regions.
This aspect of the method is particularly advantageous for epitope derived from therapeutical proteins, such as those selected from the group consisting of antibodies, coagulation factors and proteins used in replacement therapies.
Another related aspect of the present invention is therefore a therapeutical protein obtainable by this aspect of the method to increase the capacity of an (hydrophobic) epitope to bind to CD1a, CD1b and/or CD1c proteins.

Another related aspect of the present invention is a method to reduce the capacity of an (hydrophobic) epitope to bind to CD1a, CD1b and/or CD1c proteins comprising the steps of
a) providing isolated cells expressing at their surface CD1a, CD1b and/or CD1c;
b) specifically applying this epitope to these isolated cells;
c) putting NKT cells into contact with the cells of step b;
d) measuring a significant activation of the NKT cells,
e) substituting at least one hydrophobic amino acid by one (similar) non-hydrophobic amino acid, and/or selectively removing one hydrophobic amino acid from the (hydrophobic) epitope and/or replacing a post-translationally modified amino acid by the corresponding not modified amino acid, so as to generate a modified epitope;
f) applying the modified epitope to isolated cells expressing at their surface CD1a, CD1b and/or CD1c;
g) putting NKT cells into contact with the cells of step f, potentially presenting the modified epitope and
h) measuring a reduced activation of the NKT cells over the measure of step d.
Advantageously, the epitope is present in therapeutic proteins, preferably selected from the group consisting of antibodies, coagulation factors and proteins used in replacement therapies (e.g. in the treatment of Pompe, Gaucher or Fabry diseases).
Therefore, a related aspect of the present invention is a therapeutic protein obtainable by this method to reduce the capacity of an (hydrophobic) epitope to bind to CD1a, CD1b and/or CD1c proteins, this (modified) protein being for use in the treatment of a disease selected from the group consisting of allergic disease, auto-immune disease, and immune response towards biologicals.

Another related aspect of the present invention is a method to increase the capacity of a cell to present an epitope (preferably a peptidic epitope specific for CD1a, CD1b, CD1c, or for CD1d, or a MHCII epitope) comprising to add in one or both flanking region(s) of the said epitope an hydrophobic amino acid, a palmitoyl-cysteine moiety, a CD4-specific (such as RYD tripeptide) or a CD8-specific motif (such as TQQ tripeptide) or to add a cysteine amino acid (or motif, as described above) in both flanking regions of the epitope, preferably wherein the flanking region is less than 10 amino acid upstream or downstream of the epitope.

Another related aspect of the present invention is an *in vitro* method for detecting, isolating or depleting NKT cells in a blood or tissue sample comprising the steps of providing a surface comprising CD1a, CD1b and/or CD1c proteins, applying to the said surface an epitope determined to bind to the said CD1a, CD1b and/or CD1c proteins and to activate NKT cells and of applying the said blood or tissue sample to the surface comprising the said epitope.

### Detailed description of the invention

The present invention relates to peptides having the capacity to trigger the activation of NKT cells through specific binding to group 1 CD1 molecules (CD1a, CD1b and/or CD1c).

The present invention therefore allows to design new peptides encompassing epitopes presenting therapeutic properties. Indeed the capacity of peptides to bind to group 1 CD1 molecules (CD1a, CD1b and/or CD1c) can be measured so as to design vaccine agents comprising eptiopes activating NKT cells through presentation to group 1 CD1 molecules, or the capacity of peptides to bind to group 1 CD1 molecules (CD1a, CD1b and/or CD1c) can be tuned so as to modulate (increase or decrease) the activation of specific NKT cells.

The specific modulation of the activity of NKT cells allows therapeutic applications, for instance in tumors, infections associated with intracellular pathogens, auto-immune diseases, allergy or tissue-specific chronic inflammation resulting from chronic infection, and for situations characterized by chronic endoplasmic reticulum stress.

A related aspect is the production of specific NKT cells, and elements thereof, such as their T cell receptor or the nucleic acid molecule encoding it, and their use for therapeutic applications in tumors, infections associated with intracellular pathogens, auto-immune diseases, allergy or tissue-specific chronic inflammation resulting from chronic infection, and for situations characterized by chronic endoplasmic reticulum stress.

The present invention allows to design peptides or polypeptides which naturally activate NKT cells and from which such property is eliminated by aminoacid mutation, deletion or addition so as to suppress their capacity to activate NKT cells and describes the use of such modified peptides or polypeptides for therapeutic applications in which an alloantigen has to be administered, such as a coagulation factor, a therapeutic antibody, an enzyme or a viral vector for the practice of gene therapy or gene vaccination.

The present invention allows to design CAR NKT cells carrying an antibody receptor expressed at the surface of NKT cells and the design of NKTCR T cells in which the potential of alternative T cells such as CD4+ effector or regulatory T cells, or CD8+ cytotoxic cells is harnessed. The invention covers such cells and their therapeutic application in tumors or autoimmune diseases.

Another related aspect of the present invention relates to methods to isolate and expand NKT cells, methods for diagnostic purposes wherein NKT cells are used as biomarkers for disease identification and follow-up, and methods to deplete NKT cells from biological samples.

More specifically, the present invention relates to a method to identify immunogenic peptides based on their capacity to bind to group 1 CD1 molecules (CD1a, CD1b and/or CD1c) and/or on their capacity to activate NKT cells for therapeutical purposes.

Immunogenic peptides are derived from tumor-associated antigens, intracellular pathogens, autoantigens, allergens or extracellular infectious agents, and used for the treatment of the corresponding disease, in either a preventive or a suppressive setting.

Conversely, the present invention provides a method to prepare NKT cells specific for peptidic antigens associated with tumors, intracellular pathogens, autoantigens, allergens or extracellular infectious agents. The peptidic antigens are presented to isolated NKT cells by cells expressing group 1 CD1 molecules (CD1a, CD1b and/or CD1c).

The method can be adapted for the preparation, detection of depletion of NKT cells specific for a tumor, an intracellular pathogen, an autoantigen, an allergen or a extracellular infectious agent, wherein a sample comprising NKT cells is put in contact of a surface comprising a group 1 CD1 molecules (CD1a, CD1b and/or CD1c) loaded with a corresponding peptidic antigen.

The present invention also relates to methods for increasing the immunogenicity or the regulatory properties of peptides for use in the treatment of tumors and of intracellular infections, or for the therapy of autoimmune diseases, allergy or chronic inflammatory diseases, the method comprising the addition of a potentiating motif located in the flanking residues of the CD1-epitope.

### Definitions

The terms "peptide" or "peptidic" when used herein refers to a molecule comprising an amino acid sequence of more than 2 amino acids, connected by peptide bonds. In the context of the present invention, non-amino acid structures (like for example a linking organic compound) may be grafted on peptides and the term "peptide" may thus encompass a peptidic molecule further comprising a non-peptidic moiety (preferably, the peptidic moiety represents the largest part of the molecule; weight amino acids:total weight >50%). Peptides according to the invention can contain any of the conventional 20 amino acids or modified versions thereof, or can contain non-naturally occurring amino acids incorporated by chemical peptide synthesis or by chemical or enzymatic modification. The terms "peptide" or "immunogenic peptide" are used indifferently. The terms "peptide" or "protein" are used indifferently, but "protein" is preferentially used for naturally-existing long peptides (or biologicals), such as peptides used in replacement therapy in the treatment of haemophilia or Pompe, Gaucher or Fabry diseases.
The term "epitope" when used herein refers to one or, less preferably, several portions (which may define a conformational epitope) of a protein which is/are specifically recognized and bound by an antibody or a portion thereof (Fab', Fab2', etc.) or a receptor presented at the cell surface of a B or T cell lymphocyte, and which is able, by said binding, to induce an immune response.
The term "antigen" when used herein refers to a structure of a macromolecule comprising one or more hapten(s) and/or comprising one or more T cell epitopes. Typically, said macromolecule is a protein or peptide (with or without polysaccharides) or made of proteic composition and comprises one or more epitopes; said macromolecule can herein alternatively be referred to as "antigenic protein" or "antigenic peptide".
The term "flanking residues" refers to aminoacid residues, which are located outside of the core binding region of an epitope bound to Group 1 CD1 molecule and further recognized by NKT cells; in the context of the present invention, the "flanking residues" correspond to the region of between 1 and 20 amino acids (preferably between 2 and 10 amino acids) at the N- and/or the C- end of the group 1 CD1 epitope.
The term "potentiating moiety" or "potentiating motif" refers to (i) peptidic sequences added to epitopes so as to increase activation of NKT cells or to modulate NKT cell activity, and located within the flanking residues of the epitope, or to (ii) DNA or RNA sequences as agonists of TLRs, or to (iii) short alkyl sequences, or to (iv) any combination of the former.
The term "hydrophobic amino acid" preferably refers to aromatic amino acids (phenylalanine, tryptophan, tyrosine), aliphatic amino acids (valine, isoleucine, leucine, methionine), acylated amino acids, and decarboxylated amino acids such as citrulline. Other non-conventional or artificial amino acids sharing similar properties (i.e. determined, for instance according to water:octanol partition coefficient) are also encompassed by the present terminology.
The term "NKT cells" refers to cells of the innate immune system characterized by the fact that they express the master transcription factor PLZF and carry receptors such as CD162 and NKG2D. In the context of the present invention, unless explicitly mentioned differently, NKT cells recognize epitopes presented by the group 1 CD1 molecules, either CD1a, CD1b or CD1c, and are (specifically) activated upon such recognition. NKT cells express an antigen-specific receptor (CD3) capable of forming a cognate synapse with an antigen-presenting cell expressing the MHC-like CD1 restriction element. The term "NKT cell epitope" refers to a part of an antigenic protein that is specifically recognized and bound by a receptor at the cell surface of a T lymphocyte. In the context of the present invention, unless explicitly mentioned differently, a NKT cell epitope is an epitope bound by Group 1 CD1 molecules.
The "CD1 molecule" refers to a non-MHC derived molecule, or MHC-like molecule, made of 3 alpha chains and an anti-parallel set of beta chains arranged into a deep hydrophobic groove opened on one or both sides and capable of presenting lipids, glycolipids or hydrophobic peptides to NKT cells.
The term "NKTCR T cell" refers to a T cell or either NKT lineage, or of a class I (CD8+) or class II-restricted (CD4+) lineage, which is transfected or transduced with a receptor obtained from a NKT cell clone having specificity for a peptide-CD1 complex.
The term "CAR NKT cell" refers to a NKT cell which is transfected with a construct containing an antibody (or part of it including the variable chains), constant parts of the TCR with, optionally, signalling domains.
The term "immune disorders" or "immune diseases" refers to diseases wherein a reaction of the immune system is responsible for or sustains a malfunction or non-physiological situation in an organism. Immune disorders in the context of the present invention refer to pathology induced by infectious agents and tumor surveillance, autoimmune diseases, allergic diseases, and diseases resulting from chronic exposure to inflammation or endoplasmic reticulum stress conditions
The term "therapeutic biological" or "biological" refers to a protein, peptide or factor (i.e. any peptidic molecule) displaying polymorphism when compared between two individuals of the same species, or carrying epitopes capable of binding to a group 1 CD1 molecule and activate NKT cells, and, more in general, any protein, peptide or factor that induces an (alloreactive) immune response in the subject receiving the biological.

CD1 molecules are heterodimers of a heavy chain non-covalently associated with beta-2 microglobulin with a general fold similar to that of MHC class 1 molecules. They form a groove capable of binding hydrophobic residues whose architecture varies much between molecules. CD1 molecules differ much in terms of function, cell expression and intracellular trafficking, underscoring their non-redundant function.

The present invention therefore relates to methods by which hydrophobic peptidic epitopes having the capacity to bind to CD1a, CD1b or CD1c recruit and activate NKT cells. As said epitopes ensure antigen-specificity, the methods described here represent an antigen-specific approach to increase or modulate innate immunity in several therapeutic indications.
By hydrophobic epitopes, it is meant epitopes comprising a succession of hydrophobic amino acids, including post-translationally modified amino acids having such hydrophobic properties. The succession of hydrophobic amino acids may be either a linear stretch of several hydrophobic amino acids, or an hydrophobic moment, where the succession is due to the (2D or 3D) configuration of the epitope. Tools for the prediction of such hydrophobic moment do exist.

The present invention relates to hydrophobic peptides encompassing at least one CD1a-, CD1b- and/or CD1c-restricted T cell epitope.

The 3 group 1 CD1 restriction elements should be considered separately, as their general structure and expression pattern significantly differ from each other.

CD1a is expressed primarily on dendritic and skin Langerhans cells. It is present at the surface of cells and screens the extracellular environment. CD1a is endocytosed and traffics in a Rab2 - Arf6 dependent pathway to the early endosome due to the absence of a long cytoplasmic tail. It functions as a presentation molecule for antigens encountered in said early endosomes or at cell surface. The only foreign antigen known to bind to CD1a are derived from mycobacterium lipopeptides, depending on the length and the saturation state of the lipid moiety. CD1a also presents sulfatides, a class of sulfoglycolipids abundant in myelin sheets.

CD1b is expressed on dendritic cells. Its large groove can accommodate very large residues or even several lipid molecules at the same time. CD1b is ideally suited for presentation of hydrophobic moments. CD1b binds to molecular adaptors and transits via late acidic lysosomes in a LAMP1-dependent pathway. Notably, CD1b is protected during its intracellular trafficking by the MHC class II related invariant chain, much alike class II MHC molecules. CD1b molecules are involved in defense mechanisms to mycobacterium tuberculosis (mycolic acid) and possibly other antigens from bacterial origin.

CD1c is expressed on dendritic cells, Langerhans cells and B lymphocytes. It is handled by the endocytic system yet does not transit to late endosome. Its function is less well established. CD1c presents sulfatide and a number of antigens derived from bacteria and mycobacterium, as well as lipopeptides, though the peptidic sequence is protruding out of the CD1c cleft (which thus differs from the present invention, where at least one hydrophobic amino acid interferes with the CD1c cleft).

CD1 molecules present antigens by sequestring hydrophobic moieties, such as, in the present application, hydrophobic moieties presented by peptides (acyl chains grafted on amino acids, aliphatic residues of amino acids such as Leu, Ile,...), within a hydrophobic cleft composed of pockets. Such pockets are defined as A', C' and F' and vary in their architecture and solvent accessibility between CD1 molecules. The cleft of the epitope binding region of CD1 molecules is itself much variable from one group 1 CD1 molecule to the other. CD1a presents a cleft as a J-shaped format, with residue in A' being buried in a deep cavity closed on 3 sides, whilst the F' position remains open and can indeed accommodate longer structures. The cleft of the CD1b molecule is by far the largest and presents 3 hydrophobic pockets in A', C' and F', respectively. Interestingly, under acidic conditions, the cleft opens itself to accommodate structures which would otherwise not be able to do so. The cleft of the CD1c molecule presents a general A'-F' structure with two hydrophobic pockets, and open ends at both extremities.

Accordingly, the general structure of NKT cell epitopes varies according to the CD1 molecule. For binding to the CD1a a hydrophobic bulky residue such as phenylalanine should occupy the A' position followed by an aliphatic (hydrophobic) amino acid with an alkyl chain of various length and a second hydrophobic residue in position F'. The sequence can be symmetrical as loading can occur in both directions. However, the unusual J shape of CD1a precludes the binding of further aminoacid sequences on one side of the epitope. The CD1b molecule can accommodate 1, 2 or even 3 hydrophobic residues in the A' position, with various combinations of phenylalanine, tryptophan, and a long alkyl chain containing an hydrophobic residue to accommodate position C' and optionally position F'. The CD1c molecule accommodates sequences presenting the same characteristics as for CD1a.

The variation observed between structures of these various group 1 CD1 molecules makes it difficult to design motifs which would bind to group 1 CD1 clefts. However, a general view of the sequence of epitopes binding to CD1 molecules could be obtained by using algorithms accessible on line. For instance, peptides can be identified by entering a sequence on the prosite website. This should be considered as a general first step, which could be refined when results of functional assays described below are obtained. The aminoacid sequences able to activate NKT cells in the context of presentation of epitopes by CD1a, CD1b or CD1c are rather defined functionally, by their capacity to (1) (specifically) bind to the group 1 CD1 molecule, and (2) the capacity of the complex made between epitope and CD1 molecule to form a synapse with NKT cells and to activate them.

The identification of the CD1a, CD1b or CD1c-restricted epitopes is carried out by a functional assay. One suitable test consists in incubating either peptides encompassing epitopes, or the full protein from which the epitope is derived, with an antigen-presenting cell transfected with either CD1a, CD1b or CD1c. Interestingly, the absence of polymorphism of the CD1 molecule renders it easy to assess the relevance of NKT activation with only a limited number of samples.

To this end, CD1+ antigen-presenting cells are prepared from either an animal or human source. This is carried out preferably by starting from cells which do not express MHC molecules, nor MHC-like molecules. Mouse or human cell lines are transfected with constructs containing the sequence of a group 1 CD1 molecule together with a construct encoding the beta-2 microglobulin sequence. Such cells are checked for the presence of the CD1 receptor using a specific antibody coupled to a fluorochrome followed by detection of fluorescence using a fluorescence activated cell sorter. Preferably, cells to be transfected with the CD1 receptor should be a professional antigen-presenting cells, such as a dendritic cell, a macrophage or a B cell. Several examples are known in the art.

NKT cells as envisaged in the context of the present invention carry an antigen-specific receptor (CD3) made of an alpha-beta or of a gamma-delta chain association.

The NKT cells of the present invention are of different phenotypes, carrying the CD4 (CD4+ NKT cells) or the CD8 co-receptor (CD8+ NKT cells), or being double negative for such co-receptors (CD4-CD8-NKT cells). The present invention also relates to NKT cells carrying either the alpha-beta T cell receptor or the gamma-delta receptor.

The method of the present invention is adapted to activate CD8+ NKT cells and/or CD4-CD8- NKT cells so as to treat tumors or infectious diseases caused by an intracellular pathogen (cytotoxic function). Conversely, the method is adapted to activate CD4+ NKT cells so as to treat autoimmune or allergic diseases, or conditions characterized by endoplasmic reticulum stress (regulatory function). Such methods make use of various combinations of epitope sequences and of the presence or not of a potentiating motif within flanking residues (see below).

Subsets of NKT cells have recently been described but only for type 1, alpha-gal-ceramide-reactive, CD1d-restricted NKT cells. These include NKT1 (T-bet+, PLZF^{lo}, RORyt-), NKT2 (T-bet-, PLZF^{hi}, RORγt-) and NKT17 (T-bet-, PLZF^{int}, RORγt+). Although such information is lacking for cells restricted by CD1a, CD1b or CD1c, the applicant has discovered that NKT cells restricted by group 1 CD1 molecules are also diverse. Thus, in general, cells expressing CD4 exhibit regulatory properties with secretion of IL-4 and IL-10, whilst CD8+ NKT cells produce more IFN-γ and show more cytotoxicity activity, as do the double-negative NKT cells. This observation should be considered as a general observation, not meant to be restrictive.

The discovery at the basis of the present application is that such group 1 CD1-restricted NKT cells are in fact capable to recognize hydrophobic peptidic epitopes presented by either CD1a, CD1b or CD1c, and that such recognition is followed by activation of the corresponding NKT cells.

A similar though distinct observation had already be made for cells expressing CD1d, as described in patent applications WO2012069568, WO2012069572, WO2012069575 and WO2013174805. However, CD1d distinguishes itself form CD1a, CD1b and CD1c by several features, including structure, molecular interaction, cell trafficking, cell surface expression and repertoire of epitopes presented at the surface, making CD1d (single representative of group 2 CD1 molecules) fully distinct from the group 1 CD1a, CD1b and CD1c molecules.

Upon presentation of a peptidic epitope bound to CD1a, CD1b or CD1c, NKT cells are rapidly activated and secrete a number of cytokines activating other cells from both the innate and adaptive immune systems, and potentially exert a cytotoxic activity on CD1a-, CD1b- or CD1c-positive cells carrying the corresponding (or cognate) epitope, accordingly.

Peptides (of the invention) can be advantageously combined with potentiating motifs located outside of the core epitope as recognized by NKT cells so as to increase synapse formation and thereby increase activation of corresponding NKT cells.

Two types of potentiating motifs can be delineated according to the expected effect: enhanced cytotoxic activity or increased regulatory activity. Enhanced cytotoxic activity is appropriate in situations wherein target cells are of tumoral origin or infected with an intracellular pathogen. In such a case, NKT cells of the CD8+ or CD4-CD8- phenotype are preferable. Enhanced regulatory activity is appropriate in situations wherein tissue inflammation should be reduced, as in chronic autoimmune diseases, or chronic or repetitive exposure to allergens or infectious agents, as well as in situations in which the stress of the endoplasmic reticulum results in post translational alteration of intracellular constituents.

Potentiating motifs are preferably administered after covalent linkage to the CD1-restricted epitope, either to the amino-terminal end or carboxy-terminal end of the epitope, with or without linker sequences of various lengths. It should be understood that one or several of these potentiating motifs can be added to the epitope, depending on the contemplated application.

Potentiating motifs belong to either of the following categories:
(1) Addition of cysteine residues (or motif, as described above) within flanking residues, preferably on each side of the epitope, allows aggregation of the epitope protecting it from inappropriate cleavage and increasing epitope presentation;
(2) The addition of short alkyl chains such as palmitic acid or myristic acid (on reactive moieties of amino acids, such as -SH) is also a preferred possibility. Thioesters obtained by reacting palmitate and cysteine residues constitute a preferred example;
(3) The inclusion of short complementary sequences of co-receptors such as CD8 or CD4, or TIGIT is a preferred option. Examples are CDRs (complementarity determining regions) of specific antibodies for CD4, in particular those recognizing an epitope within the second extracellular domain of the CD4 molecule, and complementary sequences specific for the CD8 molecule, either its alpha or beta isoform;
(4) The addition of agonistic sequences for Toll-like receptors (TLRs), such as CpG-DNA motifs, dsDNA, ssDNA, LPS or Pam3Cys is also encompassed.

In all these settings, the activation of NKT cells forming a synapse with a cell presenting a group 1 CD1 molecule bound with an epitope is enhanced.

A specific case of a potentiating motif is the so-called thioreductase motif made of 2 cysteine residues separated by 2 aminoacids, which exerts a reducing activity on disulfide bridges in target molecules. Reduction of the disulfide bridge located in the second extracellular domain of the CD4 co-receptor triggers the formation of homodimers and polymers of CD4 resulting in significant increase in synapse duration. However, disulfide bridges susceptible to be reduced by a thioreductase motif adjacent to the CD1 epitope are also present in the CD8 cofactor molecule, in the CD3 receptor and in surface molecules expressed by NKT cells such as TIGIT.

Potentiating motifs as described above can be applied with alternative epitopes to those restricted by class 1 CD1 molecules, provided that a thioreductase motif is not used for CD1d-restricted epitopes or for a class II MHC restricted epitopes. Patent application WO2012069568 describes increased activation of specific NKT cells when combined to a thioreductase motif within flanking residues (WO2012069568), and patent application WO2008017517 describes that class II restricted epitopes containing a thioreductase motif in their flanking residues provide a signal to CD4+ T cells forcing them into a cytotoxic phenotype.

Advantageously, the immunogenic peptide further comprises and endosomal targeting sequence.

Peptides of the invention can advantageously be administered together (covalently, or not covalently-bound, by contrast to the potentiating motif described above) with an adjuvant molecule to increase the efficiency of epitope processing and to increase expression of CD1 molecules at cell surface. Examples are CpG motifs activating toll-like receptor 9 (TLR9), lipopolyssacharides or palmitoyl cys3 activating TLR4 or oligonucleotides activating TLR3. Conventional adjuvants such as aluminum hydroxide or an oil emulsion of mycobacterium such as complete Freund's adjuvant, or alternative molecules known in the art, can be added to peptides of the invention for administration in a subject.

The invention further relates to methods for obtaining or inducing populations of NKT cells as described above, said methods comprising the steps of:
(i) providing isolated natural CD3+ T cells;
(ii) contacting those cells with an immunogenic peptide comprising a T cell epitope presented by the CD1a, CD1b or CD1c molecule and, optionally, a potentiating motif, and
(iii) expanding said cells in the presence of IL-7 and IL-2/IL-15.

In a further aspect, the invention encompasses a method of identifying a population of NKT cells, said method comprising the steps of:
(i) providing isolated natural CD3+ T cells;
(ii) providing CD3+ T cells expressing the master regulator PLZF; and
(iii) determining that the T cells provided in (ii) display, compared to the T cells provided in (i), the characteristics described above.

In a further aspect, the invention encompasses a method to produce cells carrying a single NKT cell receptor specific for a peptide-CD1 complex, comprising the steps of:
(i) obtain a NKT cell clone specific for a peptide-CD1 complex;
(ii) isolate the DNA sequence encoding the variable part of the TCR of cells obtained in (i);
(iii) providing isolated CD3+ T cells;
(iv) introduce by genetic engineering the DNA obtained in (ii) in cells obtained in (iii);
(v) optionaly, introduce by genetic engineering the DNA sequence of cofactors in cells obtained in (iii).

It should be understood that the cells obtained in (iii) can belong to various lineages according to their contemplated use. Thus, cells of the NKT lineage or of the class I-restricted lineage are appropriate when a cytotoxic activity is sought for, such as for tumors or cells infected by an intracellular pathogen. Cells of the class II-restricted lineage are more advantageous when regulatory functions are sought for, with applications in autoimmune diseases, allergy, control of tissue homeostasis.

Cells produced in this aspect of the invention are called NKTCR T cells, in the sense that they carry a receptor specific for a peptide-CD1 complex which is used to transfect or transduce T cells of various origin.

In a further aspect, the invention encompasses a method to produce cells carrying an antibody recognition domain for a surface molecule carried by a target cell, comprising the steps of:
(i) obtain a B cell clone producing an antibody towards a target cell surface molecule;
(ii) isolate the DNA sequence encoding said antibody;
(iii) produce a DNA construct containing the DNA as obtained in (ii) with the DNA encoding the constant part of a TCR (CD3) with, optionally, or DNA sequences of signalling domains;
(iv) obtain a NKT cell clone specific for a peptide-CD1 complex;
(v) introduce the DNA as obtained in (iii) in cells as obtained in (iv)

It should be understood that the specificity of the antibody used to construct such a chimeric antibody receptor (CAR) NKT cell varies according to the target cell. Examples include, but are not limited to, ligands of NKG2D, oncogens or antigens expressed at the surface of the target cell as a result of endoplasmic reticulum stress response, or as a result of infection with an intracellular pathogen.

The NKT cell clone selected in (iv) can be chosen according to its phenotype and function, depending on the contemplated usage. Thus, as examples not meant to be exclusive, Tbet⁺, PLZF^{lo}, RORgt⁻ NKT cells are preferred when a cytotoxic activity towards a target cell is required, and; Tbet⁻, PLZF^{hi}, RORgt⁻ NKT cells are to be preferred in settings wherein a regulatory function is required. Applications include elimination of tumor cells and of cells with an intracellular pathogen in the first case, and autoimmune diseases, allergic diseases, chronic inflammation and conditions characterized by endoplasmic reticulum stress.

In any of the above, said tumor-associated antigens being an oncogene including mutated or overexpressed transcription factors, proto-oncogenes, virus-derived proteins, surviving factors or clonotypic determinants such as an idiotypic determinant derived from a B cell receptor.

In any of the above uses said intracellular pathogen-associated antigen may be any antigen derived from viruses, bacteria, mycobacterium or parasites with an intracellular life cycle.

In any of the above uses, said autoantigen may be associated with an organ-specific autoimmune disease, including type 1 diabetes, myasthenia gravis, thyroiditis, mutliple sclerosis, thyroiditis, celiac disease, inflammatory bowel disease, rheumatoid arthritis, pernicious anemia, uveitis, cochleitis and adenalitis.

In any of the above uses, chronic inflammatory diseases may be associated with chronic exposure to allergens or to infectious agents.

In any of the above uses, post-translational alterations of antigens due to endoplasmic reticulum stress may be associated with tumors, chronic auto-immune diseases, chronic infectious diseases, hyperstimulation, hypoxia or exposure to high concentrations of pro-inflammatory cytokines such as interferon-gamma (IFN-γ) or interleukine-1beta (IL1-β).

A further aspect of the invention is a (an in vitro) method for obtaining a population of CD1a, CD1b or CD1c-restricted NKT cells, said method comprising the steps of:
(i) providing an immunogenic peptide comprising a NKT cell epitope derived from a tumor-associated antigen, an intracellular pathogen-associated antigen, an autoantigen, an allergen, an infectious agent or an antigen modified at post-transcriptomic level and, optionally, a potentiating motif;
(ii) administering the immunogenic peptide to a subject (or to a sample comprising NKT cells); and optionally in the presence of an adjuvant;
(iii) obtaining a population of NKT cells.

Peptides and polypeptides which are the object of the present invention can be obtained using a functional assay. A cell line, preferably of human origin, preferably not expressing MHC class II molecules and preferably obtained from an antigen-presenting cell such as a dendritic cell, a macrophage or a B lymphocyte, is used for transfection with the sequence of the relevant group 1 CD1 molecule and the sequence of beta-2 microglobulin. Examples of such cells are the U937 cell line and HeLa cells. To note, the absence of significant polymorphism in group 1 CD1 molecules allows to use a single transfected cells for each of the group 1 CD1 molecules. Transfection is carried out by methods known in the art using viral vectors, electroporation and the like. Expression of the transfectant at cell surface is detected by an antibody specific for the CD1 molecule, which ensures the functionality of the molecule as surface expression requires the presence of beta-2 microglobulin. Detection of the transfected receptor is usually carried out using a fluorescene-labeled specific antibody and a cell sorter. Transfected cells are then incubated with full or partial aminoacid sequences of the antigen under consideration and potentially containing epitopes that can be presented by the transfected CD1 molecule. A population of CD3+ cells is obtained from peripheral blood or from tissue and incubated with one or several of the transfected cells. The presence of NKT cells specific for the CD1-presented epitope is then evaluated by NKT cell activation, such as thymidine incorporation or activation of NUR77, which is associated with the formation of a synapse in between NKT cells and the epitope-presenting transfected cell line.

The NKT epitope can in some examples carry posttranslation modifications, where appropriate, to mimic the conditons under which such epitopes are presented in vivo under pathological conditions.

Populations of NKT cells obtainable by the above methods are also part of the invention, as well as their use for (the manufacture of a medicament for) preventing or treating, in a subject, tumors, infections with an intracellular pathogen, autoimmune diseases, allergic diseases, chronic infections, situations wherein post-translationally modified antigens play a detrimental role.

The invention provides ways to augment the expansion and functional activity of NKT cells specific for epitopes bound to MHC-like molecules CD1a, CD1b or CD1c

Transfected cells are then incubated with a peptide encompassing the epitope to test. When epitopes are used, instead of full-length peptides comprising them, it is envisaged to use them under different formats with, for instance, addition of a potentiating motif, and/or post-translational modifications.

The potentiating motif in the above immunogenic peptides is placed either immediately adjacent to the epitope sequence within the peptide, or is separated from the NKT cell epitope by a linker. More particularly, the linker comprises an amino acid sequence of 7 amino acids or less. Most particularly, the linker comprises 1, 2, 3, or 4 amino acids. Linkers can also contain D-aminoacids or non-natural aminoacids or even small chemical structures. As the binding cleft is open both sides, except for CD1a, peptides longer than 7 aminoacids can be accommodated.

When the immunogenic peptide comprises an endosomal targeting sequence, a flanking sequence can be present between the NKT epitope and an endosomal targeting sequence and/or between the potentiating motif and an endosomal targeting sequence. More particularly a flanking sequence is a sequence of up to 10 amino acids, or of in between 1 and 7 amino acids, such as a sequence of 2 or 3 amino acids. More particularly, the flanking sequence contains hydrophobic (e.g. F, Y, W,) aminoacid residues (i.e. at least 2, 3, 4, 5, 6, or even 7, 8, 9 or 10 of the amino acids of the flanking region are hydrophobic) which are useful to stabilize the peptide into the CD1 molecule.

Cell samples as a source of NKT cells consists in PBMC, from which CD3+ T cells are extracted and purified before addition to cell cultures containing the CD1a, CD1b or CD1c transfected cells. NKT cells can then be amplified for establishing cell lines and clones for analysis, the methods and techniques being known in the art. Transfected cells incubated with the epitope or the protein from which the epitope is derived are then maintained in culture in the presence of IL-7 and various ratios of IL-2 and IL-15. Detection, expansion and characterization of NKT cells are then carried out as described above.

Cells transfected with one of the group 1 CD1 molecules can be used *in vivo* as, for instance, in immunocompromised animals reconstituted with a source of human cells. NOG mice, which lack cells of the adaptive and innate immunity are reconstituted by, for example, CD3+ T cells obtained from the peripheral blood of healthy individuals or of patients affected by a given disease (tumor, intracellular infection, see examples). CD1-transfected cells are loaded with either a protein containing a CD1-binding epitope, or a peptide encompassing a CD1-restricted epitope. Cells are then transplanted in the peritoneum of immunocompromised mice. After a few days, the peritoneum is punctured and cells recovered for detection, identification and expansion of NKT cells. Alternatively, transfected cells loaded with the protein or the corresponding epitope are directly injected IV in immunocompromised mice. The spleen is recovered after 3 to 4 days for detection and expansion of NKT cells.

Upon administration, peptides containing hydrophobic residues are taken up by antigen-presenting cells, and processed by different routes, as a function of the class of CD1 molecule involved and the cell expressing it. Thus, as examples not meant to be exhaustive, binding to surface-expressed CD1a molecule is followed by intracellular uptake and fusion with early endosome. Complexes are redirected to cell surface for interaction with NKT cells in a context dependent on the metabolic status of the cell, and in particular the existence or not of endoplasmic reticulum stress conditions, as prevails in tumors cells or cells infected with a pathogen. Binding to CD1b after endocytosis occurs in a different context in so far as CD1b can enter the late endosome pathway and its acidic conditions, in a process which is much alike that of the processing of group 2-restricted epitopes. In the late endosome compartment, hydrophobic peptides and lipids compete for the binding to the CD1b cleft. Once loaded, such CD1b molecules are directed towards cell surface for presentation and activation of NKT cells. CD1c molecules exchange epitopes in the endocytic pathway and interact with multiple intracellular bodies through adaptor proteins, yet not with late endosome. They are prone to present autoantigens, or mutated autoantigens for presentation at cell surface and NKT cell recognition.

The present invention relates to peptides made of hydrophobic residues which naturally constitute group 1 CD1 binding motifs. Aminoacid residues of said motifs are sometimes modified, usually by substitution with residues increasing the capacity to bind to CD1.

Methods to measure the binding of peptides to the group 1 CD1 molecule are derived from what is known in the art. Several methods can be used, which generally consist in either insolubilizing the CD1 molecule on a solid phase such as a polystyrene plate or, rather, to construct multimers of the CD1 molecule. The relative weak affinity of the binding makes it advantageous to use multimers of a size up to dexamers. Thus, multimers of the relevant group 1 CD1 molecule are incubated with (i.e. being part of) a peptidic sequence, which can be labeled for instance with biotin. The presence of peptide bound to CD1 multimers can then be assessed by fluorescence using fluorochrome-labeled avidin and detection by a fluorescence-activated cell sorter. Several alternatives based on the same principle are known in the art.

The methods described here above can be used to detect group 1 CD1-restricted NKT cells. Thus, multimers of a group 1 CD1 molecule are incubated with populations of lymphocytes of various origin, including from peripheral blood. A preferred method is to first isolate cells expressing the CD3 antigen receptor from a blood sample, usually obtained by incubation of the whole cell population on a surface (e.g. beads) coated with anti-CD3 antibodies. If required, a stimulation cycle can be carried out *in vitro* by incubating CD3+ cells with transfectants expressing single group 1 CD1 molecule and the protein from which the putative CD1 binding sequence has been obtained, or with the peptide encompassing such sequence. CD3+ cell expansion is then carried out, for instance in the presence of IL-7, IL-2 and IL-15. Cells obtained from this stimulation step can then be sorted again (i.e. with beads on which multimers of the CD1 molecule are insolubilized), followed if needed by one or several stimulation cycles.

The population of cells as restricted by one of the group 1 CD1 molecule can then be evaluated for markers of NKT cells, including transcription factors such as PLZF and surface molecules such as CD162 and NKG2D. Alternatively, the isolated CD3 cells are sorted for NKT markers before incubation with a group 1 CD1 molecule.

A majority of tumors cells express molecules of the CD1 lineage, spontaneously or after stimulation by inflammatory cytokines such as IL-1β or IFN-γ. The use of histone deacetylase (HDAC) inhibitors or of agents stimulating the endoplasmic reticulum stress (such as Brefeldin A, thapsigargin, 2-deoxyglucose, tunicamycin) significantly increases CD1 surface expression, providing a target for NKT cells.

The present application describes methods to identify epitopes with the capacity to bind to group 1 CD1 molecules and activate epitope-specific NKT cells. These epitopes, alone or containing a potentiating motif and, optionally, a late endosome targeting sequence are prepared by chemical synthesis and used for direct vaccination in individuals affected by a tumors.

In one aspect, the NKT-cell epitope is derived from tumor, including any peptide or polypeptide derived from: (1) oncogenes, including mutated transcription factors such as Tp53, hyperexpressed transcription factors or kinases, MAGE as identified in some melanomas; (2) proto-oncogenes, such as cyclin D1 expressed on soft tissues carcinomas such as those of the kidney or parathyroid, as well as in multiple myeloma; (3) virus-derived proteins, such as those from the Epstein-Barr virus in some carcinomas and in some Hodgkin-type lymphomas; (4) surviving factors, which are anti-apoptotic factors such as survivin or bcl2; (5) clonotypic determinants, such as idiotypic determinants derived from B cell receptor in follicular lymphomas or multiple myelomas or T cell receptor determinants in T cell malignancies.

NKT cells participate to the defense against tumors, either indirectly by producing cytokines able to boost both innate and adaptive responses to tumor cells or directly by killing tumor cells presenting cognate epitopes. Direct killing involves granzyme and perforin production and several alternative pathways of the tumor necrosis factor (TNF) superfamily. Tumors susceptible to be treated by the present invention include those expressing oncogenes as listed above.

Epitopes susceptible to be useful in the elicitation of such NKT cells are identified by the methods described above. Upon administration of such peptides, specific NKT cells are elicited, which specifically recognize and eliminate infected cells.

Thus NKT-cell epitope are advantageously derived from an intracellular pathogen. Such pathogens can be viruses, bacteria or parasites. Viruses include ssDNA, dsDNA and RNA viruses, with as examples Herpesviridae, Flaviviridae and Picornaviridae, influenza, measles and immunodeficiency viruses. Bacteria and mycobacteria include mycobacterium tuberculosis, other mycobacteria pathogenic for humans or animals, Yersiniosis, Brucella, Chlamydiae, Mycoplasma, Rickettsiae, Salmonellae and Shigellae. Parasites include Plasmodiums, Leishmanias, Trypanosomas, Toxoplasma gondii, Listeria, Histoplasma.

The phenotype of CD8+ NKT cells is in fact diverse, with some cells expressing the isoform alpha-alpha, or alpha-beta. The applicant made the discovery that a significant proportion of such cells express homodimers of the beta isoform, whereas the alpha isoform is classically considered as the binding unit for class I MHC molecule. In fact, the main functional difference between the alpha and beta isoforms is to be found in the increased capacity of the beta isoform to recruit kinases such as LCK to the synapse, thereby increasing early signaling and activation of cells. Thus the insertion in the flanking region(s) of a complementary sequence of the beta isoform represents a further selective advantage to recruit CD8+ NKT cells.
A large number of autoimmune diseases are considered in the present context, and in particular organ-specific autoimmune diseases, including type 1 diabetes, myasthenia gravis, multiple sclerosis, thyroiditis, celiac disease, adrenalitis, ocular diseases including uveitis, inner ear diseases such as cochleitis, to cite just the most frequent ones. In all these examples an important inflammatory component prevails in the affected tissue, due, *inter alia*, to activation of TLRs. Regulatory NKT cells form a synapse with inflammatory cells infiltrating the diseased organ, but can also directly interact with cells producing either an hormone (insulin, thyroid hormones) or constitutive proteins such as myelin. Such cells express restriction MHC-like molecules belonging to Group 1 CD1 lineage. Elicitation of such regulatory NKT cells therefore provides a double impact: regulation of the influx and function of cells infiltrating the target organ and restoration of normal cell functioning. Post-translational modifications of autoantigens are frequently observed in chronic autoimmune diseases. Peptides used to elicit NKT cells are advantageously modified according to the posttranslational modification observed in such diseases. Many examples of such modifications have been described, one of the most common ones being citrulination of arginine residues, addition of short alkyl chains including palmitoyl and myristoyl moieties, phosphorylation or sulfation. These modifications are considered as part of the present application.

A preferred application in the field of autoimmune diseases is therefore the elicitation of NKT cell carrying the CD4+ co-factor, known to produce IL-4 and IL-10, for instance via potentiating motifs, which would help recruiting CD4+ NKT cells, such as those including a complementary sequence for CD4 in flanking residues.

Chronic allergic asthma is dominated by accumulation of inflammatory cells at the level of bronchial walls and in peribronchial spaces. An allergen-specific control of chronic inflammation is therefore achievable by eliciting NKT cells with regulatory properties. Chronic exposure to infectious agents responsible of chronic obstructive bronchial disease would benefit from the same approach, considering that the pathology derives prominently from inflammation rather than infection itself.

Induction of regulatory NKT cells can be of interest to establish tolerance to agents used for therapeutic purposes such as the ones listed below. In such cases, sequences encompassing motif showing the capacity to bind to Group 1 CD1 molecules and activate NKT cells are used for vaccination purposes as described for chronic or recurrent allergen or infectious agents exposure.

The present invention thus covers applications in the field of chronic inflammation and for induction immune tolerance to biologicals used for therapeutic purposes via the elicitation of NKT cells carrying the CD4 cofactor, which can be elicited advantageously by using peptides containing Group 1 CD1 epitopes and potentiating motifs made of anti-CD4 complementary structure.

Chronic stress of the endoplasmic reticulum is observed in many situations without obvious signs of autoimmunity. These include hypoxia, such as that occurring after myocardial infarction or cerebral thrombosis or cerebral bleeding. The post-trauma period is also considered as dominated by endoplasmic reticulum stress. The inflammatory reaction which predominates after such events is detrimental to tissue repair. In these cases no specific autoantigen or infectious agent can directly be incriminated. The method presented here provides the opportunity to identify NKT cells reacting with such tissues and thereby design peptides useful for elicitation a regulatory NKT (CD4+) cell population. Dampening the inflammation shall accelerate and amplify tissue repair and restoration. Another example of this application is obesity, in which hypoxia due to the increase of the fat tissue mass is accompanied by hypoxia, stress response and inflammation.

Biologicals used for therapeutic purposes often induce an immune response which limits their use. The discovery at the basis of the present invention showed that most of these biologicals interact with the innate immune system and that this interaction is required to elicit an adaptive response. More particularly, biologicals express sequences capable of binding to restriction molecules of the group 1 CD1 family, resulting in recognition and activation of specific NKT cells. Elimination of the group 1 CD1 motif by aminoacid substitution, deletion of addition, or by introducing a postranslational alteration into the sequence prevents activation of NKT and the elicitation of an adaptive immune response. This results in full immune tolerance to the so-modified biological, which can then be administered on the long term with no risk of loosing efficiency.

Biologicals considered in this category include any peptide or polypeptide used for (1) replacement therapy in the setting of a gene defect or malfunction, including coagulation factor defect of haemophilia A (factor VIII) or B (factor IX), staphylokinase, tissue plasminogen activator, and gene defects associated with liver function, such as alpha-glucosidase in Pompe disease or galactosidase in Fabry's disease; (2) hormones such as insulin, growth hormone, parathormone; (3) cytokines and growth factors, including IFN-a, IFN-g, GM-CSF and G-CSF, cytokine receptors such as IL-1b receptor; (4) therapeutic antibodies used for the modulation of immune responses, including antibodies to IgE in allergic diseases, anti-CD3 (in particluar CD3d and CD3e) or anti-CD4 antibodies in graft rejection or auto-immune diseases, antibodies to cytokines such as IFN-g in the treatment of rheumatoid arthritis or alternative conditions, antibodies to checkpoint inhibitors such PD-1 and PD-1 ligand and TIGIT, or antibodies to immune cell surface antigens such as CD20; (5) antibodies used in the construction of CAR T cells or antibodies carried by genetically-modified B cells (CAR B cells) for use the treatment of tumors; (6) erythropoietin in the treatment of renal insufficiency; (7) viral vector used for gene therapy and gene vaccination, including adenovirus vectors, adeno-associated viral vectors, retrovirus-derived viral vectors. This list is not exhaustive and the person skilled in the art will realize that this concerns any peptide or polypeptide used for therapeutic purposes.

Peptides or polypeptides of said biologicals can be produced by chemical synthesis or by genetic engineering. Deletion, substitution, addition and/or post translational modifications could include a combination of the two methods, wherein a biological produced by genetic engineering is further treated by introduction of post-translational modifications.

In addition to the therapeutic potential of the present invention, peptides encompassing epitopes with the capacity to bind to Group I CD1 molecules in their natural sequence or after modification by addition of a potentiating motif, or after mutation, deletion or post-translational modification can be used to detect, isolate, expand or deplete corresponding NKT cells when appropriate. Oligomers or multimers of CD1 molecules are used in soluble or insolubilized format and are loaded *in vitro* with the peptide of interest. Oligomers or mutlimers are then incubated with a source of NKT cells susceptible to form a synapse with said peptide. The source of NKT cells include peripheral blood, cells obtained from tissues by puncture or biopsy, or from cultured cells. NKT cells obtained by this procedure can be used to unravel a mechanism of action or a potential toxicity or side effects induced upon administration to a subject.

The methodology can be used whenever it would be advantageous to eliminate NKT cells from a biological sample as, for instance, in auto-immune diseases. Cells obtained from peripheral blood are passed through a extracorporeal system on which oligomers or polymers made from CD1 molecules are insolubilized and loaded with peptides corresponding to the NKT cells one wishes to extract from blood, and cells depleted of the corresponding NKT cells are then readminsitered to the subject. The CD1 molecules can alternatively be produced with covalent attachment of the peptide or produced by genetic engineering as a single molecule with the peptide. Methods to achieve this are known in the art.

A further aspect of the present invention is a method for the production of (NK) T cells engineered to carry a T cell receptor (TCR) specific for a given epitope presented by a CD1 molecule. This method comprises the step of the identification and/or amplification of the TCR by polymerase chain reaction, introduction of the DNA encoding for such receptor into autologous T cells, preferably selected for a specific phenotype and/or a specific function. Preferably a cell of the NKT lineage carrying either CD8 or being negative for both CD4 and CD8 is used. For the therapy of autoimmune diseases, a CD4+ phenotype is preferred. However, it may be advantageous to select a T cell belonging to the class I-restricted (CD8+) lineage or to the class II-restricted (CD4+) lineage, according to the desired usage. Cells of either NKT class I or class II lineage can be further selected as for instance to carry the functionality of regulatory T cells or of suppressive cells carrying TIGIT or DNAM, or of cells with expression of receptors for ligands associated with cytotxic function, such as NKG2D. The one skilled in the art will understand that this list is not exhaustive. Methods to produce such cells are well known in the art. Taken together, such engineered cells are called NKTCR T cells.

A further aspect of the present invention is a method for the production of NKT cells carrying an antibody specific for a surface antigen of a target cell. In this case, the NKT cell is selected according to its phenotype and function and the antibody recognizes a ligand associated with endoplasmic reticulum stress conditions, oncogens of molecules associated with an intracellar pathogen. Methods to produce such cells are known in the art. Taken together, such engineered cells are referred to as CAR NKT cells.

The present invention also relates to NKT cells obtained, expanded and activated *in vitro* for re-administration to a host in order to increase his/her capacity to eliminate tumor cells or cells infected with a pathogen. As an alternative to the in vitro stimulation of NKT cells by CD1a, CD1b or CD1c positive APC, the invention also applies to methods of transfection or transduction of APC using a genetic construct capable of driving expression of the immunogenic peptide into the endosome pathway for loading onto CD1 molecule.

In particular, the invention provides ways to expand specific NKT cells, with as a consequence increased activity comprising, but not limited to:
(i) increased production of pro-inflammatory cytokines
(ii) increased contact- and soluble factor-dependent elimination of target cells with which a synapse is formed.
The result is therefore a more efficient response towards tumor cells or towards cells infected with an intracellular pathogens.

Conversely, the present invention also relates to NKT cells obtained, expanded and activated in vitro for passive administration to a host in order to increase the suppressive or regulatory function in the setting of chronic autoimmune diseases, chronic inflammation, or administration of a biological for therapeutic purposes.

In particular, the invention provides ways to expand specific NKT cells, with as a consequence increased activity comprising, but not limited to:
(i) increased production of suppressive cytokines
(ii) increased contact- and soluble factor-dependent regulation of the function of target cells with which a synapse is formed.
The result is therefore a suppression of the activation of cells involved in an autoimmune response, or in a response to chronic inflammation, or an immune response towards a biological administered for therapeutic purposes. Such suppressive activity includes the relief from endoplasmic reticulum stress in target cells with restoration of normal function, including the production of hormones such as insulin in diabetes, or myelin in demyelating diseases such as multiple sclerosis.

Of particular interest within the scope of the present invention is that methods described here can result in alterations of the number or function of cells acting downstream of NKT cells. Natural killer (NK) cells are highly dependent from help provided by NKT cells, both by the production of soluble factors such as cytokines and by cell to cell contact. This results in an increased or decreased activity of NK cells. Increased activity is desirable in settings such as tumors and infection with intracellular pathogens, wherein activated NK cells participate and amplify the cytotoxic response towards the tumor or the infected cells. Decreased activity is desirable in situations wherein NK cells play a detrimental role, such in tissue specific autoimmune diseases, including type 1 diabetes and multiple sclerosis.

The present invention also relates to the identification of NKT cells with required characteristics or properties in body fluids or organs. The method comprises identification of NKT cells by virtue of their surface phenotype, including expression of CD162, NKG2D and CD244. Cells are then contacted with NKT cell epitopes defined as peptides able to be presented by the CD1 molecule. Advantageously, multimers of CD1 molecules can be used to carry out this identification. Cells can then be expanded in vitro in the presence of IL-7 and IL-2 and/or IL-15.

The present invention covers both curative and preventive applications.

The curative potential of the present invention relates to the observation that NKT cells with suppressive or regulatory properties can alter the metabolism of the cells with which a synapse is formed and, as such, carry the potential to rescue target cells from endoplasmic reticulum stress, setting the stage for restoration of normal cell function. Examples of this are to be found in insulin-dependent diabetes and the restoration of insuline production, and in the capacity of oligodendrocytes to restore myelin production in demyelating diseases such as multiple sclerosis.

The curative potential of the invention is also illustrated in applications in infections with intracellular pathogens and in already present tumors. The elimination of the infected or the tumor cell is followed by the relase of antigens associated with the infection or with the tumor. Such antigens are taken up by antigen-presenting cells and thereby elicit a cascade of immune responses leading to eradication of the infection or of the tumor.

The present invention also covers the preventive usage of the therapy, either by active vaccination or by passive transfer of cells.

An additional advantage of the present invention is related to the very limited degree of polymorphism of group I CD1 molecules. Medicaments under the scope of the present invention can therefore be considered as universal molecules, in the sense that peptides are applicable as such to the great number of patients in need for a therapy.

NKT cell populations are advantageously obtained by either *in vitro* expansion or by genetic engineering (NKTCR T cells or CAR NKT cells). This cell population can be administered to patients who are not necessarily MHC compatible.

In situations in which more than one antigen is present in a subject, the medicament may consist in combination of peptides for direct vaccination approaches, or mixture of NKT cell preparations.

The medicament of the invention is usually, though not necessarily, a (pharmaceutical) formulation comprising as active ingredient at least one of the immunogenic peptides of the invention, a population of NKT cells for said immunogenic peptides or a gene therapeutic vector capable of expressing said immunogenic peptide. Apart from the active ingredient(s), such formulation will comprise at least one of a (pharmaceutically acceptable) diluent, carrier or adjuvant. In particular, the pharmaceutical composition of the invention is vaccines for prophylactic or therapeutic applications.

The NKT cell epitope is envisaged as being able to bind to more than one of the group 1 CD1 molecules. For instance, the structure of CD1a and CD1c are similar yet not identical, so that a peptide could bind to both CD1a and CD1c. This does not mean that the effect of NKT cell recognition and activation are identical, as the properties of CD1a and CD1c are different, in particular because of their different intracellular trafficking pathways.

The NKT cell epitope of the immunogenic peptides of the invention can correspond either to a natural epitope sequence of a protein or can be a modified version thereof, provided the modified NKT cell epitope retains its ability to bind within the CD1 cleft, similar to the natural NKT cell epitope sequence. The modified NKT cell epitope can have the same binding affinity for the CD1 protein as the natural epitope, but can also have a lowered or a higher affinity.

Preferably, the immunogenic peptides of the invention further comprise an amino acid sequence (or another organic compound) facilitating uptake of the peptide into early or late endosomes for processing and presentation within CD1 molecules.

The late endosome targeting is mediated by signals present in the cytoplasmic tail of proteins and corresponds to well-identified peptide motifs such as the dileucine-based [DE]XXXL[LI] or DXXLL motif (e.g. DXXXLL), the tyrosine-based YXXØ motif or the so-called acidic cluster motif. The symbol Ø represents amino acid residues with a bulky hydrophobic side chains (e.g. Ile, Leu). The late endosome targeting sequences allow for processing and efficient presentation of the antigen-derived NKT cell epitope by MHC-like CD1 molecules. This is of particular interest for CD1b which transits to late endosome for cargoing epitopes in the facilitated acidic environment, which alters the 3D structure and optimizes epitope exchange.

Isolated peptide sequences are tested by, for example, NKT cell biology techniques, to determine whether the peptide sequences elicit a NKT cell response. Those peptide sequences found to elicit a NKT cell response are defined as having NKT cell stimulating activity. Human NKT cell stimulating activity can further be tested by culturing NKT cells obtained from an individual sensitized to a tumor-derived antigen, to a pathogen-associated antigen, an autoantigen, an allergen, an infectious agent or a biological used for therapeutic purposes with a peptide/epitope derived from said antigens, and determining whether proliferation of NKT cells occurs in response to the peptide/epitope as measured, e.g., by cellular uptake of tritiated thymidine. Stimulation indices for responses by NKT cells to peptides/epitopes can be calculated as the maximum CPM in response to a peptide/epitope divided by the control CPM. A NKT cell stimulation index (S.I.) equal to or greater than 1.5 times the background level is considered "positive". Consequently, a SI of less than 1.5 times the background level is considered as "low". Positive results are used to calculate the mean stimulation index for each peptide/epitope for the group of peptides/epitopes tested.

The immunogenic peptides of the invention have preferably a mean NKT cell stimulation index of greater than or equal to 2.0. An immunogenic peptide having a NKT cell stimulation index of greater than or equal to 2.0 is considered useful as a prophylactic or therapeutic agent. More particularly, immunogenic peptides according to the invention have a mean NKT cell stimulation index of at least 2.5, at least 3.5, at least 4.0, or even at least 5.0. The absence of significant polymorphism of group 1 CD1 molecules greatly facilitates this step, as a single or a short number of epitopes should be recognized by NKT cells of the large majority of individuals.

Non-natural or modified NKT-cell epitopes can further optionally be tested for their binding affinity to group 1 CD1 molecules.

In order to determine optimal NKT cell epitopes by, for example, fine mapping techniques, a peptide having T cell stimulating activity and thus comprising at least one T cell epitope as determined by T cell biology techniques is modified by addition or deletion of amino acid residues at either the N- or C-terminus of the peptide and tested to determine a change in NKT cell reactivity to the modified peptide. An alternative methods to evaluate the degree of activation of NKT cells in the absence of over expansion relies on the evaluation of TCR engagement by measuring the degree of phosphorylation of NUR77, which occurs rapidly after such engagement. NKT cells can respond to synapse formation by tonic stimulation, which is measured by NUR77 phosphorylation considered to be positive if greater than 1.2 times the value obtained with control experiments. Low phosphorylation index is therefore considered as ≤ 1.2 the value of the controls.

In one aspect, methods are provided which comprise the stimulation *in vitro* of a blood sample by contacting an immunogenic peptide according to the invention with the isolated peripheral blood cells, and the expansion of the stimulated cell population, more particularly in the presence of IL-7 and various ratios of IL-2 and IL-15. The methods according to the invention have the advantage that higher numbers of NKT cells are produced and which are specific for the tumor-related antigen, the pathogen-associated antigen, the autoantigen, the allergen, the infectious agent or the biological used for therapeutic purposes.

Alternatively, NKT cells can be generated *in vivo*, i.e. by the administration of an immunogenic peptide provided herein to a subject (a human or a non-human mammal provided that the non-human mammal has antigen-presenting cells expressing (natural or exogenous) Group 1 CD1 molecules), and collection of NKT cells generated *in vivo.*

The tumor antigen-specific NKT cells obtainable by the above methods are of particular interest and are used for (the manufacture of a medicament for) the treatment of suppressing morbidity and/or mortality associated with tumors. This is of particular importance, as patients suffering from tumors are frequently immunocompromised, spontaneously or as a result of therapy, and therefore not capable to respond to direct vaccination. The pathogen-associated antigen-specific NKT cells obtainable by the above methods are also of particular interest to be used for (the manufacture of a medicament for) the treatment or the prevention in a subject of morbidity and/or mortality associated with infection with viruses, bacteria or parasites.

NKT cells specific for autoantigens in their native or post-translationally modified format are used for (the manufacture of a medicament for) the treatment of an autoimmune disease, for treating in a subject morbidity and/or mortality associated with the immune response towards the autoantigen. NKT cells specific for allergens or for antigens produced by infectious agents and to which a subject is chronically exposed, are used for (the manufacture of a medicament for) the treatment of an inflammatory reaction resulting from such chronic exposure, with its accompanying morbidity and mortality. NKT cells specific for biologicals used for therapeutic purposes are used (for the manufacture of a medicament) for the treatment of a subject having raised an immune response towards said biological, and who, in the absence of administration of such biological, suffers from morbidity and/or mortality.

For any of the above-described uses of the immunogenic peptides of the invention, the peptides can be replaced by the NKT cells. Both the use of allogeneic and autogeneic cells is envisaged.

The invention extends to populations of T cells carrying an antigen receptor specific for a peptide-CD1 complex (NKTCR T cells) and to CAR NKT cells. NKTCR T cells are constituted of a T cell of either the NKT lineage, or of T cells belonging to the class I-restricted (CD8+) or to the class II-restricted (CD4+) T cells. Those cells expressed a receptor obtained from an antigen-specific NKT cell. In addition, the invention extends to populations of NKT cells engineered to carry an antibody specific for a surface molecule expressed by a target cell (CAR NKT cells).
NKTCR T cells are preferably used for the treatment of tumors and infections with intracellular pathogens and, in this case, a cytolytic or cytotoxic phenotype is preferred, such as class I-restricted CD8+ T cells of NKT cells. For applications in settings requiring a regulatory function, such as in autoimmune diseases, allergic diseases, chronic inflammation or situations characterized by endoplasmic reticulum stress, a non-cytotoxic phenotype is preferred, such as class II-restricted CD4+ T cells, natural regulatory T cells or regulatory NKT cells. CAR NKT cells are preferably used for the treatment of tumors and infections with intracellular pathogens.

The present invention also relates to nucleic acid sequences encoding the immunogenic peptides of the present invention and methods for their use, e.g., for recombinant expression or in gene therapy. In particular, the nucleic acid sequences are capable of expressing immunogenic peptides of the invention. The immunogenic peptides of the invention may be administered to a subject in need by using any suitable gene therapy method. In any use or method of the invention for the prevention of morbidity/mortality associated with a tumor, an intracellular pathogen, an autoantigen, an allergen, a chronic exposure to infectious agents or a biological used for therapeutic purposes, immunization with an immunogenic peptide of the invention may be combined with adoptive cell transfer. When combined, said immunization, adoptive cell transfer and gene therapy can be used concurrently, or sequentially in any possible combination.

It should be clear for the one skilled in the art that the peptide or polypeptide used in gene therapy may be part of the full antigen from which the peptide or polypeptide is derived.

The medicament of the invention is usually, but not necessarily, a (pharmaceutical) formulation comprising as active ingredient at least one of the immunogenic peptides of the invention, a (population of) NKT cells specific to the immunogenic peptide, or a gene therapeutic vector capable of expressing said immunogenic peptide.

### Examples

### Example 1. Production of cell transfectants expressing group 1 CD1 molecules

HeLa cells derived from a human cervical carcinoma cell line (e.g. ATCC CCL-2 cell line) have been transfected by constructs including the sequence of the beta 2 microglobulin and of the sequence of a group 1 CD1 molecule. Co-transfection with both beta 2 microglobulin and CD1 has allowed a functional receptor at cell surface and succesfully transfected cells are therefore detected by reaction with a specific anti-CD1 antibody and expanded in culture medium.

HeLa cells express little or no class II major histocompatibility complex (MHC) molecule. When used for presentation of CD1-restricted molecules, an antibody towards class II MHC molecules can be added to the culture medium (see below for practical applications).

Expression of class I MHC molecules by HeLa cells has been switched off by using short hairpin RNA (shRNA), which enables stable knockdown during repeated cell division. Otherwise, the function of class I MHC molecules can be transiently suppressed by addition of a class I specific antibody during cell culture (see below for practical applications).

By expansion in culture and selection of cells using a specific antibody for surface expression of the CD1 molecules, cell lines expressing group 1 CD1 molecules (CD1a, CD1b or CD1c) are obtained.

### Example 2. Populations of NKT cells

As group 1 CD1 molecules are not expressed in the mouse, all experiments have therefore been carried out with cells obtained from human peripheral blood mononucleated cells (PBMCs).

Under more exceptional circumstances, such as in patients affected by a tumor, cells can also be obtained from tissue samples obtained by surgery or biopsy, or from fluid collections such as pleural or peritoneal.

PBMCs obtained on heparinized blood have been deprived of B lymphocytes by negative selection on anti-CD19 magnetic beads. The remaining cells are then positively selected on expression of the CD3 receptor. Such population contains cells restricted by class I and class II MHC, and CD1 restricted cells.

### Example 3. Production and characterization of CD1b-restricted NKT cells towards Tp53

A population of NKT cells specific to a tumor-associated transcription factor and restricted by the CD1 b molecule has been generated and its capacity to recognize and eliminate tumor cells evaluated.

Recombinant Tp53 protein is then incubated with HeLa cells transfected with the CD1b molecule for 16 h at 37°C in culture medium. The cells are then washed and incubated in the presence of human CD3+ T cells as obtained in Example 2, in the presence of IL-7, IL-2 and IL-15. After 6 days of incubation, CD3+ T cells cells are transferred to wells containing a new population of CD1b transfectant preincubated with recombinant Tp53 for a second cycle of activation in the presence of IL-2 and IL-15. The procedure is repeated for a total of 5 cycles.

The population of CD3+ expanding cells is then characterized for the presence of markers specific for the NKT lineage, including the master transcription factor PLZF. Cells are also characterized for expression of CD4 and/or CD8 co-factors. This is carried out by conventional flurorescence activated cell sorter (FACS). If required, the population of cells can be futher separated using magnetic beads into single cofactor-expressing cells (CD4+ or CD8+ cells). A population of double negative (CD4-CD8- cells) is usually obtained.

Peptides of 20-aminoacid length are produced by chemical synthesis covering the entire sequence of Tp53 with an overlap of 5 aminoacids.

HeLa cells transfected with CD1b are incubated over a period of 16 h with mixtures of such peptides, usually 5 peptides chosen from distinct regions of Tp53. The cells are then washed and incubated in the presence of the population of expanded human CD3+ T cells obtained as described above, either as a bulk population or as single populations separated according to their pattern of cofactor expression.

The proliferation of T cells is evaluated after 3 days using thymidine incorporation or detection of phosphorylated NUR77 using a specific antibody, as appropriate. Proliferating cells (or cells expressing the phosphorylated form of NUR77) are then submitted to serial dilutions and re-incubated in the presence of CD1b-transfected HeLa cells loaded with single Tp53 peptide. Depending on the proliferation rate, such experiment can be repeated several times up to the point that clonality can be assessed.

The sequence of the peptide (SEQ ID NO: 1) which contains the epitope activating NKT cells is then produced by synthesis with, in addition, a potentiating motif located within the flanking residues of such epitope. A preferred motif contains a complementary sequence for the CD8 co-factor (SEQ ID NO: 2), so as to select and expand a population of NKT cells with increased cytotoxic properties.

The peptide containing a Tp53 epitope and a complementary sequence for CD8 (SEQ ID NO: 2) are then used *in vitro* with human dendritic cells derived from circulating monocytes. Briefly, monocytes are prepared from PBMCs using magnetic beads coupled with an anti-CD14 antibody. Monocytes are then converted into dendritic cells by incubation with IL-4 and GM-CSF for 3 to 6 days, after which TNF-a is added for a further overnight incubation, according to published methods. Dendritic cells are then incubated overnight with the TP53-CD8 peptide, washed and further incubated in the presence of NKT cells obtained as described above. The proliferation of NKT cells (or rate of NUR77 phosphorylation) is taken as evidence that the epitope is presented under conditions wherein the presenting cell is expressing the entire array of MHC and MHC-like molecules. Further the experiments are repeated using full-length recombinant Tp53 to confirm that the processing of the protein results in presentation of the expected epitope.

To asses the relevance of such NKT cells in the treatment of tumors, NOG mice (having no CD3+ T cells) are engrafted with human tumor cells and the capacity of NKT cells to eliminate the tumor is assessed by passive transfer of such cells. Thus, a tumor cell is chosen as expressing high concentrations of Tp53, a frequent event in human tumor. The sequence of Tp53 in such tumor is determined by qPCR to ensure that no mutation would alter the epitope for which NKT cells are specific. If needed, the tumor can be treated with a histone deacetylase inhibitor, known to increase surface expression of CD1 molecules (see for instance Tiper IV, Cancer Immunol Immunother 65: 1411-1421, 2016). Engraftment of tumor cells (± 200,000) is made subcutaneously so as to be able to measure the size of the tumor over time. The day after NKT cells (1 to 5x10⁶) are injected IV. Mice are then followed over time with daily measurement of tumor growth and sacrificed when ethically justified. The tumor cells can be recovered and analyzed for signs of necrosis.

### Example 4. Production and characterization of CD1b-restricted NKT cells by direct expansion on human tumor cells expressing Tp53

The human breast cancer -derived cell line MDA-MB-231 carries a Arg280Lys mutation of Tp53 and expresses increased levels of the protein, is obtained from a commercial supplier (ATCC) and maintained in culture in the presence of a histone deacetylase inhibitor such as Trichostatin-A as described (Tiper IV, Cancer Immunol Immunother, ibid).

The Tp53-specific NKT cell clones obtained in in Example 3 are added to cell line cultures in the presence of IL-2 and IL-15 for a co-incubation of 6 days, after which non-adherent NKT cells are recovered and added to a fresh cell line population. Three to 5 cycles of stimulation are carried out.

Cells obtained and expanded after such cultures are characterized for expression of canonical markers of the NKT lineage, such as expresion of PLZF, and further characterized for expression of cofactors and surface molecules associated with the NKT lineage, using FACS.

In an alternative experiment the tumor cell line is co-cultured in the presence of CD3+ cells obtained from PBMCs of a patient affected by a corresponding tumor. Such CD3+ T cells are then expanded as described in Example 3 until NKT cell clones are obtained.

These experiments establish that human tumor cell lines express CD1b surface molecules associated with epitopes from Tp53 and that this expression is suffficient as to activate Tp53-specific NKT cells obtained from peripheral blood.

### Example 5. Production and characterization of CD1b-restricted NKT cells by direct expansion on human tumor cells expressing post-translational modifications of Tp53

A multitude of post-translational alterations of Tp53 have been described, including ubiquitylation. The latter is carried essentially by the MDM2 protein, an ubiquitin protein ligase, which is the main suppressor of Tp53, which itself exerts a negative feedback on MDM2. MDM2 is overexpressed in many tumor types, which results in hyperubiquitylation of Tp53. Ubiquitylation directs the protein to the proteasome and peptides from proteasome digestion are taken up by the endoplasmic reticulum and the transGolgy for cell surface expression.

An esophageal carcinoma cell line showing overexpression of MDM2 is maintained in culture.
The presence of high concentrations of ubiquitylated Tp53 is detected with a specific antibody. The carcinoma cell line is co-cultured with CD1b-restricted NKT cells as obtained in Example 3 and the activation of NKT cells evaluated by either thymidine incorporation or phosphorylation of NUR77 as described in Example 3.

MicroRNA-661 (miR-661) specifically targets MDM2 RNA. Transient transfection with miR-661of the carcinoma cell line suppresses expression of MDM2, increases Tp53 functionality and decreases Tp53 ubiquitylation, with reduced proteasome degradation, reduced presentation at cell surface in the context of CD1 b and reduced NKT cell activation.

These experiments establish that a human tumor cell line expressing increasing concentrations of a post-translationally modified (i.e. ubiquitylation) Tp53 results in increased activation of Tp53-specific NKT cells.

### Example 6. Production of NKTCR T cells

The sequence of an anti-Tp53 NKT cell clone as produced in Example 3 is determined by qPCR. The corresponding DNA is prepared and used to transfect class I-restricted CD8+ T cells, from which the original CD3 has been deleted. The transfection has been carried out using the gene pulser/Micropulser electroporation cuvettes (BIO-Rad) and methods as described for instance in Kreiter S, Journal of Immunology 2008; 180: 309-318.

The CD8+ T cell population is obtained from the peripheral blood of a patient in need for a therapy of a Tp53+ tumor, using magnetic beads coupled to specific antibodies to, first, eliminate B lymphocytes (anti-CD19 antibodies) and monocytes (anti-CD14 antibodies), followed by positive selection of CD8+ T cells with an anti-CD8 antibody.
The transfected CD8+ T cells is then capable of recognizing epitopes of Tp53 as presented by the CD1b molecule with the full cytotoxic armamentarium of class I-restricted T cells.
Such cells are then used for passive administration to the patient affected by a Tp53+ tumor.

### Example 7. Production and characterization of CD1b-restricted NKT cells for the treatment of mycobacterial infections

Macrophages constitute the principal reservoir of mycobacterium and control of infection requires specific recognition and elimination of such infected macrophages. Human macrophages are infected with a non-virulent strain of mycobacterium and incubated with a source of NKT cells, and the capacity of such NKT cells to eliminate infected macrophages is evaluated.

Macrophages have been obtained from PBMCs cultures for 5 days in RPM-1640 medium containing 20% autologous serum. Adherent cells have been collected after plating on tissue culture plates for 2h and non-adhenrent cells washed away.
A non-virulent strain of mycobacterium such as the M.tb H37Rv strain (ATCC 256) has been used to infect such macrophages. Long-term cultures of infected macrophages are established as monolayers in RPMI containing 10% of autologous serum.

Populations of infected macrophages are incubated with a source of NKT cells, namely CD3+ T cells obtained from PBMCs as described in Example 2. To note, the absence of polymorphism of group 1 CD1 molecule allows to use a single macrophage population to test NKT cells obtained from genetically unrelated individuals.

Cells proliferating in such cultures are expanded by cyclic exposure to mycobacterium-infected macrophages, following a method similar as the one described in Example 3 for Tp53. In a preferred setting, neutralizing antibodies to class I and class II MHC determinants are included over the culture period, so as to minimize expansion of the corresponding class I- or class II-restricted T cells.

When NKT cells are obtained, these are applied to CD1b-transfected cells which have been incubated with a single recombinant mycobacterium-derived protein. The early secretory antigen (ESA) is one of the most immunogenic protein of mycobacterium known to elicit both antibodies and cellular responses. Recombinant ESA is therefore used to load CD1b-transfected cells for a cycle of stimulation.
Alternatively, CD1b-transfected cells are further transfected with constructs of DNA encoding a mycobacterium-derived protein. This alternative provides the opportunity to assess proteins which are not available in recombinant format and allows to eliminate NKT cells responding to a lipid or glycolipid derived form the mycobacterium.

Further, overlapping peptides are produced recapitulating the sequence of the mycobacterium-derived protein and such peptides are applied to the CD1b transfected cells.
These methods, including number of cells and incubation times, are essentially similar to the ones reported in Example 3 for the Tp53 protein.

The end result is a population of NKT cells restricted by the CD1b molecule and specific for a peptide epitope of the mycobacterium. As described in Example 3, cells are then characterized and expanded in culture.

The corresponding CD1b-restricted epitope (SEQ ID NO: 3) is then produced by chemical synthesis together with a sequence containing CpG motifs as agonist for TLR9. Thus, an oligonucleotide (ODN) sequence (SEQ ID NO: 4) is covalently coupled at the carboxyterminal end of the peptide of SEQ ID4, so as to produce a peptide-ODN of sequence (SEQ ID NO: 3+ SEQ ID NO: 4 in one molecule).

The peptide-ODN is then used for presentation by dendritic cells prepared as described in Example 3 and cells are incubated with a preparation of CD3+ T cells obtained from peripheral blood, as described in Example 2. Activation of NKT cells is assessed either by thymidine incorporation or NUR77 phosphorylation.

To evaluate the in vivo relevance of human anti-mycobacterium NKT cells, NOG mice are infected with the mycobacterium strain and groups of mice are followed over time with evaluation of the mycobacterial load after NKT cells passive administration (usually 2x10⁵ cells per mouse).

### Example 8. Production and characterization of CD1c-restricted NKT cells specific for myelin and evaluation of their effect on myelin formation by oligodendrocytes

A human oligodendrocytic (ODN) cell line is used under various culture conditions to elicit a population of CD1c-restricted NKT cells and the capacity of such cells to modulate the production of myelin is characterized.

Human oligodendrocytic precursor cells (OPCs) (A2B5+ OPCs) are obtained from commercial suppliers. OPCs are prepared with microbeads coated with antibodies to the A2B5 antigen and to PGDFRa, so as to obtain differentiation in mature OPCs.

The OPC cell line is maintained in culture and its capacity to produce the myelin oligodendrocytic glycoprotein (MOG) is evaluated over time using a specific ELISA, or by in situ hybrization for the proteolipid protein (PLP). Parallel cultures are carried out under conditions increasing endoplasmic reticulum stress, such as, for instance, with addition of thapsigargin or tunicamycin. The OPC cell line is shown to express CD1 molecules at their surface as detected with a specific anti-CD1 antibody.

The OPC cell line is incubated with a source of NKT cells as prepared from PBMC as described in Example 2. After 3 days of incubation, non adherent cells are recovered, washed and re-incubated under the same conditions with fresh OPC cell line. The stimulation cycle is repeated 3 to 4 times. Non-adherent cells are then recovered, washed and characterized for their phenotype. In parallel experiments, incubation steps are carried out in the presence of antibodies to class I and class II molecules.

Cells recovered from such culture conditions are analyzed for the presence of markers characteristic for the NKT lineage, including expression of PLZF.

Further preparation of cells is then obtained by culture in the presence of a CD1c transfectant obtained as described in Example 1 and presenting MOG protein-derived epitopes after loading with recombinant MOG protein. Three to 4 cycles of stimulation are carried out under conditons defined in Example 3 for Tp53-specific NKT cells. The cell population obtained at the end of this stimulation cycle is analyzed for phenotypic markers characteristic of NKT cells.

The specificity for MOG is then assessed by using mixtures of peptides overlapping the sequence of the MOG protein in a progressive approach as described in Example 3.

Peptides encompassing epitopes recognized by NKT cells are produced by chemical synthesis. One peptide containing the appropriate epitope is further produced as such (SEQ ID NO: 4) or including a potentiating motif located within flanking residues and containing a sequence of the variable part of an antibody to the CD4 cofactor (end of SEQ ID NO: 6).

The full sequence of the epitope and potentiating motif is therefore SEQ ID NO: 6.

The NKT cell clones obtained as described above are used in culture with human monocyte-derived dendritic cells preloaded with the peptide of SEQ ID NO: 5or peptide containing a complementary sequence for the CD4 cofactor (SEQ ID NO: 6). After 3 to 4 cycles of stimulation carried out each time on fresh dendritic cells, the non-adherent cells are collected and characterized for their phenotype.

Clones of NKT cells are then incubated with the human OPC cell line and their capacity to restore myelin production is analyzed by in situ hybridization for PLP or major basic protein (MBP), the main constituent of myelin.

NOG mice are reconstituted with the ODN cell line followed by IV administration of NKT cell clones and the production of human myelin assessed by ELISA.

### Example 9. Production and characterization of CD1c-restricted NKT cells specific of GAD65 and evaluation of their effect on the production of insulin by Langerhans islet b cells

A human β cell line producing insulin is used under various culture conditions to elicit a population of CD1c-restricted NKT cells and the capacity of such cells to modulate the production of insulin is evaluated.

The human β cell line Blox5 (Levin F, Molecular Endocrinology, 2001 15, 478) is maintained in culture and its capacity to produce insulin is evaluated over time in a specific ELISA. Parallel cultures are carried out under conditions of increased endoplasmic reticulum stress as in Example 8 (see also Marré ML, Journal of autoimmunity 2016, 72, 33-46). The cell line is shown to express CD1 molecules at surface as detected with a specific anti-CD1 antibody.

The cell line is incubated with human PBMC as a source of NKT cells as described in Example 2, following the method as described in Example 8 for ODNs.

Cells recovered from such culture conditions are analyzed for the presence of markers characteristic for the NKT lineage, including expression of PLZF.

Such NKT cells are then incubated with a CD1c transfectant cell line as described in Example 1. In the present case, the transfectant is first incubated with GAD65 in either its full length or as mixture of 20 aminoacid-long peptides covering the entire sequence of the protein with an overlap of 5 aminoacids. Some of the epitopes are modified as to contain alkyl chains such as palmitoyl or myristoyl chains, so as to mimic the alterations of GAD65 accompanying endoplasmic reticulum stress.

The population of NKT cells obtained after 3 to 4 cycles of stimulation is analyzed for phenotypic markers.

Peptides encompassing epitopes recognized by NKT cells are produced by chemical synthesis and used in stimulation assays. One peptide encompassing an epitope restricted by CD1c (SEQ ID NO: 7), is synthesized including a potentiating motif located within flanking residues and containing a sequence of the variable part of an antibody to the CD4 cofactor, so as to obtain a full sequence of SEQ ID NO: 8. Such peptide is further modified by palmitoylation of Cys in position 2 of peptide of SEQ ID NO: 8by formation of a thioester bound (peptide of SEQ ID NO: 9), using methods known in the art.

The NKT cell clones obtained as described above are used in culture with human monocyte-derived dendritic cells preloaded with the peptide of SEQ ID NO: 9. After 3 to 4 cycles of stimulation carried out each time on fresh dendritic cells, the non-adherent cells are collected and characterized for their phenotype.

Clones of NKT cells are then incubated with the human β cell line and their capacity to restore insulin production is analyzed.

To note, in this example, the control of insulin production is under the dependence of GAD65-specific CD1c-restricted NKT cells, GAD65 being also produced by the β cell line as a surface-anchored enzyme converting glutamate into γ-aminobutyric acid (GABA), which exerts a protective effect on β cells and their capacity to produce insulin after binding to a β cell surface receptor, GABAR. Surface expression of GAD65 is assessed with a specific antibody.

NOG mice are reconstituted with the β cell line as submitted to increasing endoplasmic reticulum stress conditions, followed by IV administration of NKT cell clones and the production of human insulin is detected by ELISA.

### Example 10. Production and characterization of CD1a-restricted NKT cells specific for a house-dust mite-derived allergen, Der p 1, and evaluation of their effect on the control of asthma in an experimental mouse model made transgenic for the CD1a molecule

Bronchial asthma as defined by increased airway reactivity to inhalation of aceytylcholine is driven by inflammatory changes occurring at the bronchial epithelial level by inhalation of allergens. Epithelial cells produce a number of pro-inflammatory molecules and chemokines driving inflammation and increased airway hyperreactivity. NKT cells restricted by CD1a control such events and reduce airway hyperreactivity.

CD1a-expressing transfectant cells as obtained in Example 1 are incubated with recombinant Der p1 together with a population of human CD3+ PBMCs obtained as described in Example 2. After 3 to 4 cycles of stimulation in the presence of II-2, IL-7 and IL-15, the presence of NKT cells is evaluated by phenotype determination and expression of the canonical transcription factor, PLZF.

The population of Der p 1-specific NKT cells is then incubated with CD1a-transfectant cells preloaded with 20-aminoacid long peptides encompassing the full sequence of Der p 1, and the stimulation cycle is repeated 3 to 4 times. Clones reacting with single epitopes are then expanded.

Peptides containing a CD1a-restricted epitope are produced by synthesis. A peptide encompassing a CD1a-restricted epitope is selected (SEQ ID NO: 10) and produced together with a potentiating motif containing a thioredox motif of the format C-XH-C, wherein C stands for cysteine and H for histidine as a proton exchanger (SEQ ID NO: 11), to obtain the full sequence SEQ ID NO: 12.

A mouse strain expressing the CD1a molecule is used for developping a model of asthma, using conventional protocols. Methods for obtaining such mouse strain are known in the art.

Briefly, mice are sensitized by a single IP injection of Der p1 (100 µg) adsorbed on alum, followed one week later by 3 intranasal administrations of Der p 1 in saline (10 µg per administration). Under such conditions, mice develop an hyperreactivity to inhalation of acetylcholine as measured by body plethysmography (see for instance, Vanoirbeek JA, Toxicology Science 2014; 80: 310-321).

Such mice are reconstituted by IV administration of anti-Der p1 NKT cells (2x10⁵ cells per mouse) after IP sensitization and before intranasal administration of the allergen.

The capacity of Der p 1-specific NKT cells specific for SEQ ID NO: 12to prevent bronchial hyperreactivity as assessed by inhalation of increasing concentrations of acetylcholine is then measured in a body plethysmograph.

### Example 11. Production and characterization of CD1a-restricted NKT cells specific for an infectious agent protein, and evaluation of their effect on the control of chronic airway inflammation in an experimental mouse model made transgenic for the CD1a molecule

Haemophilus influenzae (HI) is a small Gram-negative coccobacillus, which can cause chronic colonization of the airways and thereby constitutes a significant aggravating factor for patients affected by chronic obstructive pulmonary disease (COPD), leading to chronic inflammation and tissue destruction. This inflammation is often exacerbated in the presence of influenza virus and is thought to result primarily from an innate immune response.

CD1a-restricted type B HI (Hlb)-specific NKT cells are developped and evaluated for the control of bronchial and lung inflammatory infiltrates.

CD1a-expressing transfectant cells as obtained in Example 1 are incubated with the 26-kDa outer membrane protein of Hlb (OMP26), a highly conserved antigen among HI types, and cultured in the presence of a population of human CD3+ cells obtained from PBMCs as described in Example 2. After 3 to 4 cycles of stimulation, the population of CD3+ T cells is assessed for markers of the NKT lineage, including expression of the PLZF transcription factor.

Peptides encompassing the full sequence of OMP26 are obtained with an averaged length of 20 aminoacid and an overlap of 5 aminoacids. These peptides are used to load CD1a transfectant cells, which are further cultured in the presence of the NKT cell population as obtained above. After 3 to 4 cycles of stimulation, NKT cells are characterized for surface phenotype, cytokine production and transcriptome.

The peptide of sequence (SEQ ID NO: 13) is activating such CD1a-restricted NKT cells and produced by synthesis with one cysteine inserted into flanking residues at both the amino- and carboxyterminal ends of the epitope (SEQ ID NO: 14).

A mouse strain (C57BL/6) expressing the CD1a molecule as described in Example 10 is used to evaluate the capacity of NKT cells specific to OMP26 to control airway inflammation. Mice are inoculated by intranasal (IN) application of a low pathogenicity influenza virus (A/PR8) followed 3 days later by IN application of 10⁵ CFU of Hlb. The extent of inflammation is measured in airways by evaluating infiltrates of inflammatory cells in lungs and bronchial tree.

Such mice are reconstituted with OMP26-specific CD1a-restricted NKT cells, prior to, or 1 day after, IN application of Hlb and the effect of cell administration is evaluated on lung and bronchial tree inflammation. It is demonstrated that such reconstitution alleviates signs of chronic inflammatory cell infiltration.

### Example 12. Acidic α glucosidase (AAG) is rendered non-immuno-genic by elimination of CD1b-restricted epitopes

The acidic α glucosidase (AAG) is functionally missing in Pompe's disease, which leads to early death in the infantile form of the disease, or progressive accumulation of glycogen and muscle dysfunction in late onset disease. However, administration of the recombinant form of AAG is accompanied by an immune response which precludes further use of the enzyme.

CD1b-transfectant cells as produced in Example 1 are incubated overnight at 37°C with the recombinant form of human AAG (SEQ ID NO: 15), washed and cultured in the presence of human CD3+ cells obtained from PBMCs as described in Example 2. After 3 to 4 cycles of stimulation, the CD3+ T cells are retrieved from the culture, washed an evaluated for surface markers and expression of PLZF.

Peptides covering the full 952 aminoacids sequence of AAG are synthesized with a length of 20 aminoacids and an overlap of 5 aminoacids. These peptides are used for loading CD1b transfectant cells, which are further incubated with the population of NKT cells as obtained above. Proliferating NKT cells are expanded on peptide-loaded CD1b transfectant cells and their phenotype evaluated.

Peptides encompassing epitopes presented by CD1b are mutated so as to identify the aminoacids required for such presentation, as assessed by loss of proliferation of the corresponding NKT cell clones. The required mutations are then applied to the full protein.
The capacity of the mutated protein to prevent activation of NKT cells is checked by loading CD1b transfectant cells and incubation with NKT cell clones as described above.

The mutated recombinant AAG of SEQ ID NO: 16is tested for its activity.

### Example 13. Vaccination with a class II-restricted epitope of the adeno-associated viral (AAV) vector eliminates the adaptive immune response towards the vector and allows efficient gene therapy

Preimmunization with a viral vector T cell epitope containing a potentiating motif prevents subsequent development of CD4+ effector T cells towards viral vector proteins.

The AAV vector capsid, which consists in 3 proteins (VP1, -2 and -3), is commonly used for the practice of gene therapy. However, such vectors elicit antibodies and cytotoxic immune responses, which severely restricts their use. Both antibodies and cytotoxic CD8+ cells are dependent on activation of class II-restricted CD4+ T cells, indicating that preventing CD4+ T cell activation would lead to a safe and easier practice of gene therapy.

Mice are treated by IV administration of 5 µl containing 2x10¹¹ AAV vector particles and the spleen is retrieved ten days after. CD4+ T cells are prepared form the splenocyte population by a combination of methods known in the art, involving magnetic beads coated with anti-CD4 antibodies. Peptides covering the entire 735 aminoacid sequence of the VP1 capsid protein, are produced by chemical synthesis, with a length of 20 aminoacids and an overlap of 5 aminoacids. Such peptides are then loaded on dendritic cells taken as antigen-presenting cells, followed by addition of the splenocyte CD4+ population. Co-cultures are carried out over 10 days in the presence of cytokines to expand AAV-specific class II-restricted CD4+ effector cells. Three cycles of stimulation are carried out with fresh dendritic cells loaded with peptides.

A peptide (SEQ ID NO: 17) encompassing an epitope activating CD4+ T cells is confirmed to be specific by evaluating thymidine incorporation as a marker of proliferation. The peptide of interest is then produced by chemical synthesis together with a potentiating motif made of a cysteine residue added to flanking regions on each side of the epitope (SEQ ID NO: 18).

This peptide (SEQ ID NO: 18) is administered 4 times by the subcutaneous route and at fortnight intervals into mice together with alum as an adjuvant. The spleen of such mice is obtained 10 days after the last immunization and the presence of CD4+ T cells to the AAV capsid is evaluated in a functional assay.

It is shown that CD4+ T cells obtained by this immunisation schedule exert a regulatory activity characterized by suppression of antigen presentation and control of activation of bystander CD4+ T cells belonging to both the class II-restricted repertoire and the CD1d restricted repertoire. Apoptosis of target antigen-presenting cells, as well a bystander activated T cells, results from a combination of regulatory mechanisms, including TIGIT-PRV, TRAIL-DR4 and FasL-Fas interactions, this list not being exhaustive. To note, this suppressive active is exerted on the response towards the ensemble of CD4+ T cell epitopes of the AAV capsid due to the combination of suppressive properties on antigen-presenting cells and on bystander CD4+ T cells activated at the sruface of the antigen-presenting cells.

Mice immunized with peptide of SEQ ID NO: 18are then used for direct IV administration of 10⁹ AAV vector particles administered twice at an interval of 6 weeks. It is shown that such mice do not mount an immune response towards the AAV vector, even after a boost injection of AAV particles.

### Example 14. Production and characterization of class II-restricted regulatory CD4+ T cells specific for transglutaminase in celiac disease

Celiac disease is an autoimmune disease resulting from the conversion of glutamine contained in gliadin, a component of gluten, into glutamic acid carried out by type 2 transglutaminase. Class II-restricted epitopes containing such glutamic acid residues elicit the activation of class II-restricted CD4+ T cells when presented by the DQ2 or DQ8 restriction element. Gluten-specific CD4+ T cells leads to the production of high concentrations of anti-gluten antibodies and symptoms of celiac disease. Eliminating CD4+ T cells specific for gluten would represent a cure for celiac disease.

Naïve human CD4+ T cells are obtained from peripheral blood of subjects carrying either the DQ2 or DQ8 haplotype using microbeads coated with an anti-CD4+ antibody. Such cells are incubated with autologous dendritic cells derived from peripheral blood monocytes and loaded with peptide of sequence (SEQ ID NO: 19) so as to obtain effector CD4+ T cells.

In parallel experiments, a peptide of same sequence but containing a potentiating motif within flanking residues is used. Thus, a peptide of sequence (SEQ ID NO: 20) is produced, which contains in its flanking residues a sequence derived from an antibody to CD4, specific for the second extracellular domain of CD4.

Addition of the peptide of sequence (SEQ ID NO: 20) to dendritic cells in cultures containing class II-restricted effector CD4+ T cells results in the conversion of effector CD4+ T cells into cells with strong regulatory activity. This activity includes induction of apoptosis of the antigen-presenting dendritic cell and that of bystancer effector CD4+ T cells activated at the surface of the same dendritic cell.

Gluten-specific regulatory CD4+ T cells are therefore shown to suppress an ongoing immune response towards gluten and are therefore of use for the treatment of celiac disease.

### Example 15. Production and characterization of CD1d-restricted CD8+ NKT cells for vaccination towards Dengue virus infection

Infection with Dengue virus can result in severe reactions, in particular when the subject is exposed to a second virus serotype, a phenomenon known as antibody-dependent enhancement (ADE). Thus, a strategy which would favor the elicitation of cells with cytotoxic properties towards infected cells, instead of antibodies, has the potential to be safer.

The flavivirus non-structural glycoprotein NS1 protein contains epitopes potentially presented by the CD1d molecule. In order to identify them, synthetic peptides covering the entire sequence of the protein are produced, with an overlap of 5 aminoacids. Such peptides are then added for an overnight incubation to human dendritic cells obtained by conversion of peripheral blood monocytes, as detailed in Example 5. Loaded dendritic cells are then incubated in the presence of CD3+ lymphocytes obtained from PBMCs (see Example 2). Cultures are carried out in the presence of antibodies towards class I and class II molecules so as to prevent activation of corresponding cells.

After 3 to 4 stimulation cycles, the CD3+ T cells are recovered and analyzed in terms of phenotype and expression of the canonical transcription factor, PLZF. Cells are then further expanded and cloned.

Peptide of SEQ ID NO: 21 is produced by synthesis with inclusion in its flanking residues of a complementary sequence for the CD8 co-factor and used in vitro to stimulate NKT cells by presentation with dendritic cells. The additon of a CD8 cofactor complementary sequence forces NKT towards a cytotoxic phenotype, which eliminates the dendritic cell with which a synapse has been formed.

This peptide is therefore considered for use for vaccination in individuals susceptible to contract the Dengue infection, and in particular for individuals already exposed to a first Dengue serotype and who are therefore at risk of severe systemic complications.

## Claims

1. A method to determine the capacity of a peptidic epitope to selectively bind to CD1a, CD1b and/or CD1c proteins comprising the steps of :
a) providing isolated cells expressing at their surface CD1a, CD1b and/or CD1c;
b) selectively applying the said epitope to the said isolated cells;
c) putting NKT cells into contact with the cells of step b potentially presenting the said epitope;
d) and measuring the activation level of the said NKT cells.

2. The method of claim 1, wherein the epitope is from an isolated protein or a synthetic peptide comprising the amino acid sequence of a protein or a fragment thereof, the said protein being from a tumor, an intracellular pathogen, an auto antigen a biological protein, an allergen or an agent causing chronic inflammation and/or wherein the epitope comprises post-translationally modified amino acids, preferably wherein the hydrophobicity of the said post-translationally modified amino acid is higher than the corresponding non modified amino acid.

3. A peptide obtainable by the method according to any one of the preceding claims for use in vaccination against cancer, against infections with intracellular pathogens, against auto-immune diseases or against allergic diseases.

4. A method to increase the capacity of an epitope to bind to CD1a, CD1b and/or CD1c proteins comprising the steps of
a) providing isolated cells expressing at their surface CD1a, CD1b and/or CD1c;
b) specifically applying the said epitope to the said isolated cells;
c) putting NKT cells into contact with the cells of step b;
d) measuring a low or no activation of the said NKT cells;
e) modifying the said epitope by adding a moiety selected from the group consisting of an hydrophobic amino acid, an hydrophobic post-translationally modified amino acid residue and/or deleting a non-hydrophobic amino acid present in the said epitope, so as to generate a modified protein;
f) specifically applying the said modified epitope to the said isolated cells;
g) putting NKT cells into contact with the cells of step f, potentially presenting the said modified epitope and
h) selecting a modified epitope showing an increased activation of the said NKT cells in step g over the measure of step d.

5. The method of claim 4, wherein the NKT cells are CD8+ or CD4-/CD8- and preferably wherein the epitope is derived from cancerous cells or from cells infected with an intracellular pathogen.

6. The method of claims 4 or 5 further comprising the step of adding in one or both flanking region(s) of the epitope a palmitoyl-cysteine moiety, a CD8-specific binding moiety, a TLR agonist and/or to add a cysteine amino acid or a cysteine motif in both flanking regions.

7. A peptide obtainable by the method of claim 5 or 6 for use in the treatment or vaccination against the cancer or the intracellular pathogen.

8. The method of claim 4, wherein the NKT cells are CD4+ and preferably wherein the epitope is derived from an autoantigen, an allergen or an agent causing chronic inflammation, and a therapeutical protein selected from the group consisting of antibodies, coagulation factors and proteins used in replacement therapies,.

9. The method according of claim 8, further comprising the step of adding in one or both flanking region(s) of the said epitope a palmitoyl-cysteine moiety, a CD4-specific binding moiety, a TLR agonist and/ or to add a cysteine amino acid or a cystyeine motif in both flanking regions.

10. A therapeutical protein obtainable by the method of claim 8 or 9.

11. A method to reduce the capacity of an epitope to bind to CD1a, CD1b and/or CD1c proteins comprising the steps of
a) providing isolated cells expressing at their surface CD1a, CD1b and/or CD1c;
b) specifically applying the said epitope to the said isolated cells;
c) putting NKT cells into contact with the cells of step b;
d) measuring a significant activation of the said NKT cells,
e) substituting at least one hydrophobic amino acid by one (similar) non-hydrophobic amino acid, and/or selectively removing one hydrophobic amino acid from the said epitope and/or replacing a post-translationally modified amino acid by the corresponding not modified amino acid;
f) specifically applying the modified epitope to the said isolated cells;
g) putting NKT cells into contact with the cells of step f, potentially presenting the said modified epitope and
h) selecting a modified epitope showing a reduced activation of the said NKT cells in step g, over the measure of step d.

12. The method of claim 11, wherein the epitope is derived from a therapeutical protein, selected from the group consisting of antibodies, coagulation factors and proteins used in replacement therapies.

13. The therapeutical protein obtainable by the method of claim 12 for use in the treatment of a disease selected from the group consisting of allergic disease and auto-immune disease.

14. A method to increase the capacity of a cell to present an epitope comprising to add in one or both flanking region(s) of the said epitope an hydrophobic amino acid, a palmitoyl-cysteine moiety, a CD4-specific motif, a CD8-specific motif or to add a cysteine amino acid or a cysteine motif in both flanking regions.

15. An *in vitro* method for detecting, isolating or depleting NKT cells in a blood or tissue sample comprising the steps of providing a surface comprising CD1a, CD1b and/or CD1c proteins, applying to the said surface an epitope determined to bind to the said CD1a, CD1b and/or CD1 c proteins and to activate NKT cells and of applying the said blood or tissue sample to the surface comprising the said epitope.
